# EUROPEAN PATENT SPECIFICATION

(11) **EP 2 129 801 B1**
(45) Date of publication and mention of the grant of the patent: **06.08.2014**
(21) Application number: 08748870.6
(22) Date of filing: 04.04.2008
(51) Int. Cl.: C12Q 1/68

(54) **TBC1D1 AS A DIAGNOSTIC MARKER FOR OBESITY OR DIABETES**
TBC1D1 ALS DIAGNOSTISCHER MARKER FÜR FETTLEIBIGKEIT ODER DIABETES
TBC1D1 COMME MARQUEUR DIAGNOSTIQUE DE L'OBESITE OU DU DIABETE

(30) Priority: 04.04.2007 EP 07007072
(43) Date of publication of application: 09.12.2009
(73) Proprietor: Deutsches Diabetes-Zentrum Träger: Deutsche Diabetes Forschungs- gesellschaft e.V., 40225 Düsseldorf (DE)
(72) Inventor: AL-HASANI, Hadi, 51467 Bergisch Gladbach (DE); JOOST, Hans-Georg, 14532 Kleinmachnow (DE); KLUGE, Reinhart, 14558 Nuthetal (DE); LEICHT, Katja, 14473 Postdam (DE); CHADT, Alexandra, 40215 Düsseldorf (DE)
(74) Representative: Krauss, Jan
(86) International application number: PCT/EP2008/002713
(87) International publication number: WO 2008/122422

(56) References cited:
- WO-A-02/099049
- US-A1- 2003 096 782
- STONE STEVEN ET AL: "TBC1D1 is a candidate for a severe obesity gene and evidence for a gene/gene interaction in obesity predisposition." HUMAN MOLECULAR GENETICS, vol. 15, no. 18, 7 August 2006 (2006-08-07), pages 2709-2720, XP002445674 ISSN: 0964-6906 cited in the application
- DATABASE UniProt [Online] 3 October 2003 (2003-10-03), "TBC1 domain family member 1." XP002445690 retrieved from EBI accession no. UNIPROT:Q86TI0 Database accession no. Q86TI0
- DATABASE UniProt [Online] 3 October 2003 (2003-10-03), "TBC1 domain family member 1." XP002445691 retrieved from EBI accession no. UNIPROT:Q60949 Database accession no. Q60949
- ALEXANDRA CHADT ET AL: 'Tbc1d1 mutation in lean mouse strain confers leanness and protects from diet-induced obesity' NATURE GENETICS vol. 40, no. 11, 01 November 2008, pages 1354 - 1359, XP055010041 DOI: 10.1038/ng.244 ISSN: 1061-4036
- JOOST HANS-GEORG: "The genetic basis of obesity and type 2 diabetes: lessons from the new zealand obese mouse, a polygenic model of the metabolic syndrome.", RESULTS AND PROBLEMS IN CELL DIFFERENTIATION 2010 LNKD- PUBMED:20865367, vol. 52, 2010, pages 1-11, ISSN: 0080-1844

### SEQUENCE LISTING

<110> Deutsches Institut für Ernährungsforschung
<120> A method for diagnosing obesity or diabetes
<130> D60210PCT
<150> EP 07007072.7
   <151> 2007-04-04
<160> 102
<170> PatentIn version 3.4
<210> 1
   <211> 1168
   <212> PRT
   <213> Homo sapiens
<400> 1
   Met Glu Pro Ile Thr Phe Thr Ala Arg Lys His Leu Leu Ser Asn Glu
   1               5                   10                  15
   Val Ser Val Asp Phe Gly Leu Gln Leu Val Gly Ser Leu Pro Val His
               20                  25                  30
   Ser Leu Thr Thr Met Pro Met Leu Pro Trp Val Val Ala Glu Val Arg
           35                  40                  45
   Arg Leu Ser Arg Gln Ser Thr Arg Lys Glu Pro Val Thr Lys Gln Val
       50                  55                  60
   Arg Leu Cys Val Ser Pro Ser Gly Leu Arg Cys Glu Pro Glu Pro Gly
   65                  70                  75                  80
   Arg Ser Gln Gln Trp Asp Pro Leu Ile Tyr Ser Ser Ile Phe Glu Cys
                   85                  90                  95
   Lys Pro Gln Arg Val His Lys Leu Ile His Asn Ser His Asp Pro Ser
               100                 105                 110
   Tyr Phe Ala Cys Leu Ile Lys Glu Asp Ala Val His Arg Gln Ser Ile
           115                 120                 125
   Cys Tyr Val Phe Lys Ala Asp Asp Gln Thr Lys Val Pro Glu Ile Ile
       130                 135                 140
   Ser Ser Ile Arg Gln Ala Gly Lys Ile Ala Arg Gln Glu Glu Leu His
   145                 150                 155                 160
   Cys Pro Ser Glu Phe Asp Asp Thr Phe Ser Lys Lys Phe Glu Val Leu
                   165                 170                 175
   Phe Cys Gly Arg Val Thr Val Ala His Lys Lys Ala Pro Pro Ala Leu
               180                 185                 190
   Ile Asp Glu Cys Ile Glu Lys Phe Asn His Val Ser Gly Ser Arg Gly
           195                 200                 205
   Ser Glu Ser Pro Arg Pro Asn Pro Pro His Ala Ala Pro Thr Gly Ser
       210                 215                 220
   Gln Glu Pro Val Arg Arg Pro Met Arg Lys Ser Phe Ser Gln Pro Gly
   225                 230                 235                 240
   Leu Arg Ser Leu Ala Phe Arg Lys Glu Leu Gln Asp Gly Gly Leu Arg
                   245                 250                 255
   Ser Ser Gly Phe Phe Ser Ser Phe Glu Glu Ser Asp Ile Glu Asn His
               260                 265                 270
   Leu Ile Ser Gly His Asn Ile Val Gln Pro Thr Asp Ile Glu Glu Asn
           275                 280                 285
   Arg Thr Met Leu Phe Thr Ile Gly Gln Ser Glu Val Tyr Leu Ile Ser
       290                 295                 300
   Pro Asp Thr Lys Lys Ile Ala Leu Glu Lys Asn Phe Lys Glu Ile Ser
   305                 310                 315                 320
   Phe Cys Ser Gln Gly Ile Arg His Val Asp His Phe Gly Phe Ile Cys
                   325                 330                 335
   Arg Glu Ser Ser Gly Gly Gly Gly Phe His Phe Val Cys Tyr Val Phe
               340                 345                 350
   Gln Cys Thr Asn Glu Ala Leu Val Asp Glu Ile Met Met Thr Leu Lys
           355                 360                 365
   Gln Ala Phe Thr Val Ala Ala Val Gln Gln Thr Ala Lys Ala Pro Ala
       370                 375                 380
   Gln Leu Cys Glu Gly Cys Pro Leu Gln Ser Leu His Lys Leu Cys Glu
   385                 390                 395                 400
   Arg Ile Glu Gly Met Asn Ser Ser Lys Thr Lys Leu Glu Leu Gln Lys
                   405                 410                 415
   His Leu Thr Thr Leu Thr Asn Gln Glu Gln Ala Thr Ile Phe Glu Glu
               420                 425                 430
   Val Gln Lys Leu Arg Pro Arg Asn Glu Gln Arg Glu Asn Glu Leu Ile
           435                 440                 445
   Ile Ser Phe Leu Arg Cys Leu Tyr Glu Glu Lys Gln Lys Glu His Ile
       450                 455                 460
   His Ile Gly Glu Met Lys Gln Thr Ser Gln Met Ala Ala Glu Asn Ile
   465                 470                 475                 480
   Gly Ser Glu Leu Pro Pro Ser Ala Thr Arg Phe Arg Leu Asp Met Leu
                   485                 490                 495
   Lys Asn Lys Ala Lys Arg Ser Leu Thr Glu Ser Leu Glu Ser Ile Leu
               500                 505                 510
   Ser Arg Gly Asn Lys Ala Arg Gly Leu Gln Glu His Ser Ile Ser Val
           515                 520                 525
   Asp Leu Asp Ser Ser Leu Ser Ser Thr Leu Ser Asn Thr Ser Lys Glu
       530                 535                 540
   Pro Ser Val Cys Glu Lys Glu Ala Leu Pro Ile Ser Glu Ser Ser Phe
   545                 550                 555                 560
   Lys Leu Leu Gly Ser Ser Glu Asp Leu Ser Ser Asp Ser Glu Ser His
                   565                 570                 575
   Leu Pro Glu Glu Pro Ala Pro Leu Ser Pro Gln Gln Ala Phe Arg Arg
               580                 585                 590
   Arg Ala Asn Thr Leu Ser His Phe Pro Ile Glu Cys Gln Glu Pro Pro
           595                 600                 605
   Gln Pro Ala Arg Gly Ser Pro Gly Val Ser Gln Arg Lys Leu Met Arg
       610                 615                 620
   Tyr His Ser Val Ser Thr Glu Thr Pro His Glu Arg Lys Asp Phe Glu
   625 630 635 640
   Ser Lys Ala Asn His Leu Gly Asp Ser Gly Gly Thr Pro Val Lys Thr
   645 650 655
   Arg Arg His Ser Trp Arg Gln Gln Ile Phe Leu Arg Val Ala Thr Pro
   660 665 670
   Gln Lys Ala Cys Asp Ser Ser Ser Arg Tyr Glu Asp Tyr Ser Glu Leu
   675 680 685
   Gly Glu Leu Pro Pro Arg Ser Pro Leu Glu Pro Val Cys Glu Asp Gly
   690 695 700
   Pro Phe Gly Pro Pro Pro Glu Glu Lys Lys Arg Thr Ser Arg Glu Leu
   705 710 715 720
   Arg Glu Leu Trp Gln Lys Ala Ile Leu Gln Gln Ile Leu Leu Leu Arg
   725 730 735
   Met Glu Lys Glu Asn Gln Lys Leu Gln Ala Ser Glu Asn Asp Leu Leu
   740 745 750
   Asn Lys Arg Leu Lys Leu Asp Tyr Glu Glu Ile Thr Pro Cys Leu Lys
   755 760 765
   Glu Val Thr Thr Val Trp Glu Lys Met Leu Ser Thr Pro Gly Arg Ser
   770 775 780
   Lys Ile Lys Phe Asp Met Glu Lys Met His Ser Ala Val Gly Gln Gly
   785 790 795 800
   Val Pro Arg His His Arg Gly Glu Ile Trp Lys Phe Leu Ala Glu Gln
   805 810 815
   Phe His Leu Lys His Gln Phe Pro Ser Lys Gln Gln Pro Lys Asp Val
   820 825 830
   Pro Tyr Lys Glu Leu Leu Lys Gln Leu Thr Ser Gln Gln His Ala Ile
   835 840 845
   Leu Ile Asp Leu Gly Arg Thr Phe Pro Thr His Pro Tyr Phe Ser Ala
   850 855 860
   Gln Leu Gly Ala Gly Gln Leu Ser Leu Tyr Asn Ile Leu Lys Ala Tyr
   865 870 875 880
   Ser Leu Leu Asp Gln Glu Val Gly Tyr Cys Gln Gly Leu Ser Phe Val
   885 890 895
   Ala Gly Ile Leu Leu Leu His Met Ser Glu Glu Glu Ala Phe Lys Met
   900 905 910
   Leu Lys Phe Leu Met Phe Asp Met Gly Leu Arg Lys Gln Tyr Arg Pro
   915 920 925
   Asp Met Ile Ile Leu Gln Ile Gln Met Tyr Gln Leu Ser Arg Leu Leu
   930 935 940
   His Asp Tyr His Arg Asp Leu Tyr Asn His Leu Glu Glu His Glu Ile
   945 950 955 960
   Gly Pro Ser Leu Tyr Ala Ala Pro Trp Phe Leu Thr Met Phe Ala Ser
   965 970 975
   Gln Phe Pro Leu Gly Phe Val Ala Arg Val Phe Asp Met Ile Phe Leu
   980 985 990
   Gln Gly Thr Glu Val Ile Phe Lys Val Ala Leu Ser Leu Leu Gly Ser
   995 1000 1005
   His Lys Pro Leu Ile Leu Gln His Glu Asn Leu Glu Thr Ile Val
   1010 1015 1020
   Asp Phe Ile Lys Ser Thr Leu Pro Asn Leu Gly Leu Val Gln Met
   1025 1030 1035
   Glu Lys Thr Ile Asn Gln Val Phe Glu Met Asp Ile Ala Lys Gln
   1040 1045 1050
   Leu Gln Ala Tyr Glu Val Glu Tyr His Val Leu Gln Glu Glu Leu
   1055 1060 1065
   Ile Asp Ser Ser Pro Leu Ser Asp Asn Gln Arg Met Asp Lys Leu
   1070 1075 1080
   Glu Lys Thr Asn Ser Ser Leu Arg Lys Gln Asn Leu Asp Leu Leu
   1085 1090 1095
   Glu Gln Leu Gln Val Ala Asn Gly Arg Ile Gln Ser Leu Glu Ala
   1100 1105 1110
   Thr Ile Glu Lys Leu Leu Ser Ser Glu Ser Lys Leu Lys Gln Ala
   1115 1120 1125
   Met Leu Thr Leu Glu Leu Glu Arg Ser Ala Leu Leu Gln Thr Val
   1130 1135 1140
   Glu Glu Leu Arg Arg Arg Ser Ala Glu Pro Ser Asp Arg Glu Pro
   1145 1150 1155
   Glu Cys Thr Gln Pro Glu Pro Thr Gly Asp
   1160 1165
<210> 2
   <211> 1266
   <212> PRT
   <213> Mus musculus
<400> 2
   His Val Phe Leu Ala Leu Gln Thr Ser Pro Arg Met Glu Ala Ile Thr
   1 5 10 15
   Phe Thr Ala Arg Lys His Pro Phe Pro Asn Glu Val Ser Val Asp Phe
   20 25 30
   Gly Leu Gln Leu Val Gly Ser Leu Pro Val His Ser Leu Thr Thr Met
   35 40 45
   Pro Met Leu Pro Trp Val Val Ala Glu Val Arg Arg Leu Ser Gly Gln
   50 55 60
   Cys Ser Lys Lys Glu Pro Arg Thr Lys Gln Val Arg Leu Trp Val Ser
   65 70 75 80
   Pro Ser Gly Leu Arg Cys Glu Pro Asp Leu Glu Lys Ser Gln Pro Trp
   85 90 95
   Asp Pro Leu Ile Cys Ser Ser Ile Phe Glu Cys Lys Pro Gln Arg Val
   100 105 110
   His Lys Leu Ile His Asn Ser His Asp Pro Ser Tyr Phe Ala Cys Leu
               115                 120                 125
   Ile Lys Glu Asp Ala Ala His Arg Gln Ser Leu Cys Tyr Val Phe Lys
       130                 135                 140
   Ala Asp Asp Gln Thr Lys Val Pro Glu Ile Ile Ser Ser Ile Arg Gln
   145                 150                 155                 160
   Ala Gly Lys Ile Ala Arg Gln Glu Glu Leu Arg Cys Pro Ser Glu Phe
                   165                 170                 175
   Asp Asp Thr Phe Ala Lys Lys Phe Lys Val Leu Phe Cys Gly Arg Val
               180                 185                 190
   Thr Val Ala His Lys Lys Ala Pro Pro Ala Leu Ile Asp Glu Cys Ile
           195                 200                 205
   Glu Lys Phe Asn His Val Ser Cys Gly Arg Arg Thr Asp Trp Glu Ala
       210                 215                 220
   Pro Thr Gly Gln Pro Ser Ala Pro Gly Pro Arg Pro Met Arg Lys Ser
   225                 230                 235                 240
   Phe Ser Gln Pro Gly Leu Arg Ser Leu Ala Phe Arg Lys Glu Phe Gln
                   245                 250                 255
   Asp Ala Ser Leu Arg Ser Ser Thr Phe Ser Ser Phe Asp Asn Asp Ile
               260                 265                 270
   Glu Asn His Leu Ile Gly Gly His Asn Val Val Gln Pro Thr Asp Met
           275                 280                 285
   Glu Glu Asn Arg Thr Met Leu Phe Thr Ile Gly Gln Ser Glu Val Tyr
       290                 295                 300
   Leu Ile Ser Pro Asp Thr Lys Lys Ile Ala Leu Glu Lys Asn Phe Lys
   305                 310                 315                 320
   Glu Ile Ser Phe Cys Ser Gln Gly Ile Arg His Val Asp His Phe Gly
                   325                 330                 335
   Phe Ile Cys Arg Glu Cys Ser Gly Gly Gly Ser Gly Gly Phe His Phe
               340                 345                 350
   Val Cys Tyr Val Phe Gln Cys Thr Asn Glu Ala Leu Val Asp Glu Ile
           355                 360                 365
   Met Met Thr Leu Lys Gln Ala Phe Thr Val Ala Ala Val Gln Gln Thr
       370                 375                 380
   Ala Lys Ala Pro Ala Gln Leu Cys Glu Gly Cys Pro Leu Gln Gly Leu
   385                 390                 395                 400
   His Lys Leu Cys Glu Arg Ile Glu Gly Met Asn Ser Ser Lys Thr Lys
                   405                 410                 415
   Leu Glu Leu Gln Lys His Leu Thr Thr Leu Thr Asn Gln Glu Gln Ala
               420                 425                 430
   Thr Ile Phe Glu Glu Val Gln Lys Leu Arg Pro Arg Asn Glu Gln Arg
           435                 440                 445
   Glu Asn Glu Leu Ile Ile Ser Phe Leu Arg Cys Leu Tyr Glu Glu Lys
       450                 455                 460
   Gln Lys Glu His Ser His Thr Gly Glu Pro Lys Gln Thr Leu Gln Val
   465                 470                 475                 480
   Ala Ala Glu Asn Ile Gly Ser Asp Leu Pro Pro Ser Ala Ser Arg Phe
                   485                 490                 495
   Arg Leu Asp Ser Leu Lys Asn Arg Ala Lys Arg Ser Leu Thr Glu Ser
               500                 505                 510
   Leu Glu Ser Ile Leu Ser Arg Gly Asn Lys Ala Arg Gly Leu Gln Asp
           515                 520                 525
   His Ser Ala Ser Val Asp Leu Asp Ser Ser Thr Ser Ser Thr Leu Ser
       530                 535                 540
   Asn Thr Ser Lys Glu Leu Ser Met Gly Asp Lys Glu Ala Phe Pro Val
   545                 550                 555                 560
   Ser Glu Thr Ser Phe Lys Leu Leu Gly Ser Ser Asp Asp Leu Ser Ser
                   565                 570                 575
   Asp Ser Glu Gly His Ile Ala Glu Glu Ser Ala Leu Leu Ser Pro Gln
               580                 585                 590
   Gln Ala Phe Arg Arg Arg Ala Asn Thr Leu Ser His Phe Pro Val Glu
           595                 600                 605
   Cys Pro Ala Pro Pro Glu Pro Ala Gln Ser Ser Pro Gly Val Ser Gln
       610                 615                 620
   Arg Lys Leu Met Arg Tyr His Ser Val Ser Thr Glu Thr Pro His Glu
   625                 630                 635                 640
   Arg Asn Val Asp His Leu Pro Gly Gly Glu Ser Gln Gly Cys Pro Gly
                   645                 650                 655
   Gln Pro Ser Ala Pro Pro Pro Pro Arg Leu Asn Pro Ser Ala Ser Ser
               660                 665                 670
   Pro Asn Phe Phe Lys Tyr Leu Lys His Asn Ser Ser Gly Glu Gln Ser
           675                 680                 685
   Gly Asn Ala Val Pro Lys Ser Val Ser Tyr Arg Asn Ala Leu Arg Lys
       690                 695                 700
   Lys Leu His Ser Ser Ser Ser Val Pro Asn Phe Leu Lys Phe Leu Ala
   705                 710                 715                 720
   Pro Val Asp Glu Asn Asn Thr Cys Asp Phe Lys Asn Thr Asn Arg Asp
                   725                 730                 735
   Phe Glu Ser Lys Ala Asn His Leu Gly Asp Thr Asp Gly Thr Pro Val
               740                 745                 750
   Lys Thr Arg Arg His Ser Trp Arg Gln Gln Ile Phe Leu Arg Val Ala
           755                 760                 765
   Thr Pro Gln Lys Ala Cys Asp Ser Pro Ser Arg Tyr Glu Asp Tyr Ser
       770                 775                 780
   Glu Leu Gly Glu Leu Pro Pro Arg Ser Pro Leu Glu Pro Val Cys Glu
   785                 790                 795                 800
   Asp Gly Pro Phe Gly Pro Val Gln Glu Glu Lys Arg Lys Thr Ser Arg
                   805                 810                 815
   Glu Leu Arg Glu Leu Trp Lys Lys Ala Ile Leu Gln Gln Ile Leu Leu
               820                 825                 830
   Leu Arg Met Glu Lys Glu Asn Gln Lys Leu Gln Ala Ser Glu Asn Asp
   835 840 845
   Leu Leu Asn Lys Arg Leu Lys Leu Asp Tyr Glu Glu Ile Thr Pro Cys
   850 855 860
   Leu Lys Glu Val Thr Thr Val Trp Glu Lys Met Leu Ser Thr Pro Gly
   865 870 875 880
   Arg Ser Lys Ile Lys Phe Asp Met Glu Lys Val His Ser Ala Val Gly
   885 890 895
   Gln Gly Val Pro Arg His His Arg Gly Glu Ile Trp Lys Phe Leu Ala
   900 905 910
   Glu Gln Phe His Leu Lys His Pro Phe Pro Ser Lys Gln Gln Pro Lys
   915 920 925
   Asp Val Pro Tyr Lys Glu Leu Leu Lys Lys Leu Thr Ser Gln Gln His
   930 935 940
   Ala Ile Leu Ile Asp Leu Gly Arg Thr Phe Pro Thr His Pro Tyr Phe
   945 950 955 960
   Ser Ala Gln Leu Gly Ala Gly Gln Leu Ser Leu Tyr Asn Ile Leu Lys
   965 970 975
   Ala Tyr Ser Leu Leu Asp Gln Glu Val Gly Tyr Cys Gln Gly Leu Ser
   980 985 990
   Phe Val Ala Gly Ile Leu Leu Leu His Met Ser Glu Glu Glu Ala Phe
   995 1000 1005
   Lys Met Leu Lys Phe Leu Met Phe Asp Met Gly Leu Arg Lys Gln
   1010 1015 1020
   Tyr Arg Pro Asp Met Ile Ile Leu Gln Ile Gln Met Tyr Gln Leu
   1025 1030 1035
   Ser Arg Leu Leu His Asp Tyr His Arg Asp Leu Tyr Asn His Leu
   1040 1045 1050
   Glu Glu His Glu Ile Gly Pro Ser Leu Tyr Ala Ala Pro Trp Phe
   1055 1060 1065
   Leu Thr Val Phe Ala Ser Gln Phe Pro Leu Gly Phe Val Ala Arg
   1070 1075 1080
   Val Phe Asp Met Ile Phe Leu Gln Gly Ser Glu Val Ile Phe Lys
   1085 1090 1095
   Val Ala Leu Ser Leu Leu Gly Ser His Lys Pro Leu Ile Leu Gln
   1100 1105 1110
   His Glu Asn Leu Glu Thr Ile Val Asp Phe Ile Lys Asn Thr Leu
   1115 1120 1125
   Pro Asn Leu Gly Leu Val Gln Met Glu Lys Thr Ile Ser Gln Val
   1130 1135 1140
   Phe Glu Met Asp Ile Ala Lys Gln Leu Gln Ala Tyr Glu Val Glu
   1145 1150 1155
   Tyr His Val Leu Gln Glu Glu Leu Ile Glu Ser Ser Pro Leu Ser
   1160 1165 1170
   Asp Asn Gln Arg Met Glu Lys Leu Glu Lys Thr Asn Ser Ser Leu
   1175 1180 1185
   Arg Lys Gln Asn Leu Asp Leu Leu Glu Gln Leu Gln Val Ala Asn
   1190 1195 1200
   Ala Arg Ile Gln Ser Leu Glu Ala Thr Val Glu Lys Leu Leu Thr
   1205 1210 1215
   Ser Glu Ser Lys Leu Lys Gln Ala Ala Leu Thr Leu Glu Val Glu
   1220 1225 1230
   Arg Ser Ala Leu Leu Gln Met Val Glu Glu Leu Arg Arg Gln Ser
   1235 1240 1245
   Ala Arg Pro Ser Thr Pro Glu Pro Asp Cys Thr Gln Leu Glu Pro
   1250 1255 1260
   Thr Gly Asp
   1265
<210> 3
   <211> 5688
   <212> DNA
   <213> Homo sapiens
<400> 3
   tgctcctggt gccgccaccg tccgccggtg cctgttgctg ccgccgccgc gggacctgct     60
   gtgtcctcag ctgggtggag aagaggcggg cgccgagccg aggggagccc cctccccgtc    120
   cccccgcggc gggaagagcg cagccagccg ggtgcgatgg actccccgcc cgcccaggcc    180
   gtccccagga tgcccccaag cacctgcgcg tcccggcccg gccccgggct ctgagcgcgc    240
   cgcggcacag gtttctgcat atgaagtgtg taaaatagat tgcttgatcc aaaacagaaa    300
   aacagtgata actgttttgc tgagttccca gacccttccc aagatggaac caataacatt    360
   cacagcaagg aaacatctgc tttctaacga ggtctcggtg gattttggcc tgcagctggt    420
   gggctccctg cctgtgcatt ccctgaccac catgcccatg ctgccctggg ttgtggctga    480
   ggtgcgaaga ctcagcaggc agtccaccag aaaggaacct gtaaccaagc aagtccggct    540
   ttgcgtttca ccctctggac tgagatgtga acctgagcca gggagaagtc aacagtggga    600
   tcccctgatc tattccagca tctttgagtg caagcctcag cgtgttcaca aactgattca    660
   caacagtcat gacccaagtt actttgcttg tctgattaag gaagacgctg tccaccggca    720
   gagtatctgc tatgtgttca aagccgatga tcaaacaaaa gtgcctgaga tcatcagctc    780
   catccgtcag gcggggaaga tcgcccggca ggaggagctg cactgcccgt ccgagttcga    840
   cgacacgttt tccaagaagt tcgaggtgct cttctgcggc cgcgtgacgg tggcgcacaa    900
   gaaggctccg ccggccctga tcgacgagtg catcgagaag ttcaatcacg tcagcggcag    960
   ccgggggtcc gagagccccc gccccaaccc gccccatgcc gcgcccacag ggagccagga   1020
   gcctgtgcgc aggcccatgc gcaagtcctt ctcccagccc ggcctgcgct cgctggcctt   1080
   taggaaggag ctgcaggatg ggggcctccg aagcagcggc ttcttcagct ccttcgagga   1140
   gagcgacatt gagaaccacc tcattagcgg acacaatatt gtgcagccca cagatatcga   1200
   ggaaaatcga actatgctct tcacgattgg ccagtctgaa gtttacctca tcagtcctga   1260
   caccaaaaaa atagcattgg agaaaaattt taaggagata tccttttgct ctcagggcat   1320
   cagacacgtg gaccactttg ggtttatctg tcgggagtct tccggaggtg gcggctttca   1380
   ttttgtctgt tacgtgtttc agtgcacaaa tgaggctctg gttgatgaaa ttatgatgac   1440
   cctgaaacag gccttcacgg tggccgcagt gcagcagaca gctaaggcgc cagcccagct   1500
   gtgtgagggc tgccccctgc aaagcctgca caagctctgt gagaggatag agggaatgaa   1560
   ttcttccaaa acaaaactag aactgcaaaa gcacctgacg acattaacca atcaggagca   1620
   ggcgactatt tttgaagagg ttcagaaatt gagaccgaga aatgagcagc gagagaatga 1680
   attgattatt tcttttctga gatgtttata tgaagagaaa cagaaagaac acatccatat 1740
   tggggagatg aagcagacat cgcagatggc agcagagaat attggaagtg aattaccacc 1800
   cagtgccact cgatttaggc tagatatgct gaaaaacaaa gcaaagagat ctttaacaga 1860
   gtctttagaa agtattttgt cccggggtaa taaagccaga ggcctgcagg aacactccat 1920
   cagtgtggat ctggatagct ccctgtctag tacattaagt aacaccagca aagagccatc 1980
   tgtgtgtgaa aaggaggcct tgcccatctc tgagagctcc tttaagctcc tcggctcctc 2040
   ggaggacctg tccagtgact cggagagtca tctcccagaa gagccagctc cgctgtcgcc 2100
   ccagcaggcc ttcaggaggc gagcaaacac cctgagtcac ttccccatcg aatgccagga 2160
   acctccacaa cctgcccggg ggtccccggg ggtttcgcaa aggaaactta tgaggtatca 2220
   ctcagtgagc acagagacgc ctcatgaacg aaaggacttt gaatccaaag caaaccatct 2280
   tggtgattct ggtgggactc ctgtgaagac ccggaggcat tcctggaggc agcagatatt 2340
   cctccgagta gccaccccgc agaaggcgtg cgattcttcc agcagatatg aagattattc 2400
   agagctggga gagcttcccc cacgatctcc tttagaacca gtttgtgaag atgggccctt 2460
   tggcccccca ccagaggaaa agaaaaggac atctcgtgag ctccgagagc tgtggcaaaa 2520
   ggctattctt caacagatac tgctgcttag aatggagaag gaaaatcaga agctccaagc 2580
   ctctgaaaat gatttgctga acaagcgcct gaagctcgat tatgaagaaa ttactccctg 2640
   tcttaaagaa gtaactacag tgtgggaaaa gatgcttagc actccaggaa gatcaaaaat 2700
   taagtttgac atggaaaaaa tgcactcggc tgttgggcaa ggtgtgccac gtcatcaccg 2760
   aggtgaaatc tggaaatttc tagctgagca attccacctt aaacaccagt ttcccagcaa 2820
   acagcagcca aaggatgtgc catacaaaga actcttaaag cagctgactt cccagcagca 2880
   tgcgattctt attgaccttg ggcgaacctt tcctacacac ccatacttct ctgcccagct 2940
   tggagcagga cagctatcgc tttacaacat tttgaaggcc tactcacttc tagaccagga 3000
   agtgggatat tgccaaggtc tcagctttgt agcaggcatt ttgcttcttc atatgagtga 3060
   ggaagaggcg tttaaaatgc tcaagtttct gatgtttgac atggggctgc ggaaacagta 3120
   tcggccagac atgattattt tacagatcca gatgtaccag ctctcgaggt tgcttcatga 3180
   ttaccacaga gacctctaca atcacctgga ggagcacgag atcggcccca gcctctacgc 3240
   tgccccctgg ttcctcacca tgtttgcctc acagttcccg ctgggattcg tagccagagt 3300
   ctttgatatg atttttcttc agggaacaga ggtcatattt aaagtggctt taagtctgtt 3360
   gggaagccat aagcccttga ttctgcagca tgaaaaccta gaaaccatag ttgactttat   3420
   aaaaagcacg ctacccaacc ttggcttggt acagatggaa aagaccatca atcaggtatt   3480
   tgaaatggac atcgctaaac agttacaagc ttatgaagtt gagtaccacg tccttcaaga   3540
   agaacttatc gattcctctc ctctcagtga caaccaaaga atggataaat tagagaaaac   3600
   caacagcagc ttacgcaaac agaaccttga cctccttgaa cagttgcagg tggcaaatgg   3660
   taggatccaa agccttgagg ccaccattga gaagctcctg agcagtgaga gcaagctgaa   3720
   gcaggccatg cttaccttag aactggagcg gtcggccctg ctgcagacgg tggaggagct   3780
   gcggcggcgg agcgcagagc ccagcgaccg ggagcctgag tgcacgcagc ccgagcccac   3840
   gggcgactga cagctctgca ggagagattg caacaccatc ccacactgtc caggccttaa   3900
   ctgagaggga cagaagacgc tggaaggaga gaaggaagcg ggaagtgtgc ttctcaggga   3960
   ggaaaccggc ttgccagcaa gtagattctt acgaactcca acttgcaatt cagggggcat  4020
   gtcccagtgt tttttttgtt gtttttagat actaaatcgt cccttctcca gtcctgatta   4080
   ctgtacacag tagctttaga tggcgtggac gtgaataaat gcaacttatg ttttcttgtt   4140
   ggttcctttt tgagtgtcac tgtgtttgta aagagcattc acaatacggt ggaatttcaa   4200
   aagctggaag agctcgagat catgcctcag gcaaaggcgt gggtccatcg ttcttccgag   4260
   agggtttgtg tggcgactac accctcagcg tccctggcaa ggtgcagttg gctctcgccc   4320
   attcttgtta tggaaaccta agatgatcat tgggaagatc agtgatcttg ggtcattgat   4380
   ccctggctca gaggatagcg gtttccatca taaaccaaga tgatgagttc agcctttatc   4440
   cctcgtggtt ccactagatg taacttaaag gagttaacat ttgaggactt tgttctacat   4500
   cagattttac tatttgaatg tttaagatca ctttattgaa tttgaagatc atcaaattaa   4560
   ataaaatgat ttatttaatt tggatatcct gatcactgtc aagtgaaatg gatctctctc   4620
   tttggtattt aaggaagttt gtctttaaaa aaaaaataga gtgttttcat acatttttgc   4680
   ttatcccata agtacagttg atcaaagtca tagtaggtaa atgctttatg ggacagctga   4740
   caccttttag accctaccag gtattgctag catgtgagct gcagttgtgg ggtctgagat   4800
   atttctttgt ggtagtttca tacccatact atagagtcat gtatttattt ttgcctgttg   4860
   tgtgatgtaa tgcaatcatg ttcctttgag tctccatccc ttggaaatct gacttcttgc   4920
   agaaggagta ggcacatcaa gatattcagg ggtgccccaa gagtctggga ctttcaaaaa   4980
   aaaaagatca ggctgaaact gcagtcagat ttatgacagc tgacagtttt tcagaggtcg   5040
   cacacagtga ctctcctctc tcaggatgac gaggacctgt gccttcaaca agcaaaatgc   5100
   tgctcacggt tgtcctgctt gcagccagtc actgtgtaaa gcctctctga tgtgcactta   5160
   agagtgggtt gctttctcac aaagatgggg ttctgtgcag tcacaggtca cttccttgac 5220
   aacacaatca tttctgatct ttatcactgt aaccacgtct tctattccat aggagtttct 5280
   tttgattctc tcagttgcgg ggggcatctc ttaatcctgg ggtaaaagga gagattgcca 5340
   tacttagact cactgtgagt ctccccggcc atttcacgag gagaccacag tgctgccacc 5400
   agtgcctaaa caggtggctg gcattcgaga cttcctcctg ttccctgggt cagaggatag 5460
   cggtttccat cataaaccaa gatgatgagt tcagccttta tccctcgtgg ttccgctaga 5520
   tgtaacttat aggagttaac atttgaggac tttgttctgc atcagatctt actatttgaa 5580
   tgtttactgt tggattttgg gcatcttatt actgttactc aaaaacattg actctgcatc 5640
   aagaaagaaa caagaaagca ataaaacaag aaataattca tgctcaca 5688
<210> 4
   <211> 6465
   <212> DNA
   <213> Mus musculus
<400> 4
   ggccagagat gggtttagta gaggctaatc cgcggagggc acttcatccc gggtgtttct 60
   ctgagtcccc gattttgctg cctggagtgc cagcgtccca tcctgcttcg gttcctggtt 120
   ccccccacca gcccccatcc tgccattcga ccccaatgca agaaggatga ggcgggggaa 180
   gggctggtgg ctgggtggcc tgcgggtacg ctgcgcagtg atctccgggt ccgtttcgga 240
   gggtgcgtgc agagaagcag gatccctcag ggagcgcggg ctccgctgcc acctgctgag 300
   aacctcgcac gccaaaacag cctcgggcag ggctacctgc tataaataag cggtcaagag 360
   cccggtgtgt gaccacacat cccgccggga gggccataat cacttgtgag cacgcaggga 420
   atattccggg ccaggacttc gcgctgaggc tggatggggt ggcgatgctt gcaccttccc 480
   cgagctctcg tggggtggcg cctggtggcc cctgggcggg ctgaggatgg accgctgggg 540
   agcgagggga gggaggccgc ggaggacccg cactgcagca gctgttgctc gcctgtgact 600
   cgccagtctg ggccgagtcg cctagccaga gtcgcttggt gcggagggga agggagggca 660
   tctactcctg ttcttggctc ccgggggact cactagggag attccggctg ctgtccccct 720
   cccccgcctc tgctttcccc agcgctcgcc cagcccggga ggaggaaacg cgagcgagcc 780
   gcgcgcagac acctcctcct cctcctcagc tgctgtcgcc gccgccggcc acaccgcggt 840
   gcctgttgct gcaagccacc tcggaccgac cgtctcttca acccgcaaga gaagcagaga 900
   ctgtggagtg gacccccacc ccagcagcgt ggggcggcaa gagcacagcc agctgaggac 960
   catggacgcc ccgcccgccc aggccatctt ctgggtgccc gggagcacct gcgcgcccta 1020
   gctcagcccg gagttctgag cgtgccacct cactggattc tgccctcaga agcacgtgaa 1080
   atagatcact tcatttaaag taggaaaata gtagcacgtt ttcctggctc tccagacctc 1140
   tcccaggatg gaggcaatca cattcacagc gaggaagcat ccgttcccta acgaagtctc 1200
   cgtggacttc ggcttgcagc tggtgggctc cttacccgtg cattctctca ccactatgcc 1260
   catgctgccg tgggtggtag ccgaggtacg aagactcagt ggccagtgct ctaaaaagga 1320
   gcctaggaca aagcaagtcc gcctttgggt ttcgccctcc ggactgcggt gcgagcctga 1380
   cctggagaaa agtcaaccat gggacccgct catctgttcc agcatctttg agtgcaagcc 1440
   tcagcgtgtg cacaaactga ttcacaacag tcacgacccg agctactttg cttgcctcat 1500
   taaggaggat gctgcgcaca ggcagagcct ctgctatgtg tttaaagcag acgatcaaac 1560
   aaaagtgcct gagatcatca gctccatccg gcaggccggg aagattgccc gccaggaaga 1620
   gctgcgttgc ccctccgagt tcgacgatac cttcgccaaa aagttcaagg tgctcttctg 1680
   tggccgggtg actgtggctc acaagaaggc cccacccgca ctgattgacg agtgtatcga 1740
   gaagttcaac catgtgagct gtggtcgcag aacggactgg gaagcgccca ccgggcagcc 1800
   atcagcgcct ggccccaggc ccatgcgcaa atccttctca cagcctggac tgcgctcgct 1860
   ggccttcagg aaggagttcc aggacgctag cctccgcagt agcaccttta gctcctttga 1920
   caatgacata gagaaccacc tcatcggtgg gcacaatgtg gttcagccca cagacatgga 1980
   ggagaaccga actatgctgt tcacgattgg ccaatctgaa gtttacctca tcagtcctga 2040
   caccaaaaag attgcactgg agaaaaattt taaggagata tccttttgct ctcagggcat 2100
   cagacatgtg gaccactttg gattcatctg ccgagagtgc tcgggtggcg gcagtggcgg 2160
   ctttcatttt gtctgttacg tgttccagtg cacaaatgaa gctctggttg acgagatcat 2220
   gatgactctg aagcaggctt tcacggtagc tgcggtgcag cagacggcta aggcaccagc 2280
   ccagctctgt gagggctgcc ccttgcaagg cctgcacaag ctctgcgaaa ggatagaggg 2340
   aatgaattca tctaaaacca aattagaact ccagaagcac ttgaccacac tgaccaatca 2400
   ggagcaggcc accatattcg aggaggttca gaaattgaga ccaagaaacg agcagcgaga 2460
   gaatgaatta attatttctt ttctgaggtg cttatatgaa gagaagcaaa aagagcacag 2520
   ccacactggg gagccaaagc agacactaca ggtggcagca gagaatattg ggagtgacct 2580
   gccacccagt gctagccggt tcaggttaga ttcgctgaag aacagagcaa agaggtcctt 2640
   aacagagtcc ctagagagca ttctgtcccg gggtaataaa gccagaggcc tgcaggacca 2700
   ttccgccagt gtggatctgg acagctccac ttctagtact ctaagtaaca ccagcaaaga 2760
   gctgtccatg ggtgacaagg aggccttccc cgtctctgag acctccttca agctccttgg 2820
   ctcctcagat gacctgtcca gtgactcaga gggccacatt gcagaagagt ctgccctgtt 2880
   gtcaccccag caggcgttca gaaggagagc caacaccctg agtcatttcc cagtagagtg 2940
   ccctgcgcct ccagaacctg cccagagctc tccaggggtc tctcaaagga aactcatgcg 3000
   gtaccactcc gtgagcacag agacgcctca tgaacgcaat gtggaccatc ttcctggggg 3060
   tgagtctcag ggctgcccag ggcagccttc agctccgcct ccacctcgtc ttaacccctc 3120
   cgcctcctca ccaaacttct ttaagtacct aaaacacaat tccagtggag agcaaagtgg 3180
   gaatgctgtg ccaaaaagtg tctcctaccg taatgccctg cgaaaaaaac tccattcttc 3240
   ctcctctgtg ccaaattttc taaaatttct ggctcccgta gatgaaaata acacctgtga 3300
   ctttaagaac acgaacaggg actttgaatc caaagcaaac cacctgggtg acacagatgg 3360
   gacccccgtg aagacccggc ggcactcgtg gagacagcag atattccttc gagtggccac 3420
   tccacagaag gcttgtgact ccccgagcag atatgaagat tattccgagc tgggagagct 3480
   ccctccacgc tcccctttag aaccggtgtg tgaggacggc ccatttggcc cagtacagga 3540
   agaaaagagg aagacgtcac gcgagcttcg agagctgtgg aaaaaggcca tcttgcagca 3600
   gatcctgctg ctcaggatgg agaaggagaa tcagaagcta caagcctctg aaaacgattt 3660
   gctgaacaaa cgcctcaagc ttgactatga agaaatcact ccgtgtctta aagaagtcac 3720
   tacagtgtgg gaaaagatgc ttagcactcc aggaagatcc aaaattaagt ttgacatgga 3780
   aaaagtgcac tcagctgttg ggcaaggtgt gccacgtcat caccgaggtg agatctggaa 3840
   atttctagct gagcagttcc accttaaaca cccatttcct agtaaacagc agccaaagga 3900
   cgtgccctac aaagagctcc tgaagaagct gacctcgcag cagcacgcca ttctcatcga 3960
   cctcgggcga acctttccaa cacatccata cttctctgcc cagcttggag caggtcagct 4020
   gtcactttac aacattctga aggcctactc gcttctggac caggaggttg gatactgcca 4080
   aggtctcagc tttgtggcag gcattttgct tcttcacatg agtgaggaag aggcgttcaa 4140
   gatgctcaag ttcctgatgt ttgacatggg gctgcggaaa cagtatcggc cagacatgat 4200
   tattttgcag atccagatgt accagctgtc acggctcctc cacgattacc accgagacct 4260
   ctacaaccac ctggaagagc acgagatcgg ccccagcctc tacgcggctc cctggtttct 4320
   caccgtgttc gcctcacagt tcccactcgg ctttgtagcc agagtctttg atatgatctt 4380
   ccttcaggga tcagaggtca tatttaaagt agctttaagt cttttgggga gccataagcc 4440
   cttgattcta cagcatgaga acctggaaac catcgtggac ttcataaaga acacactccc 4500
   caacctgggc ctggtgcaga tggagaagac catcagtcag gtgtttgaga tggacatcgc 4560
   caagcagctc caggcctatg aggtcgagta ccacgtgctc caggaggagc ttattgagtc 4620
   ctcgcctctc agtgacaacc aaagaatgga gaaattggag aaaaccaaca gcagcttgcg 4680
   caaacagaac cttgacctcc tggagcagtt gcaggtggca aatgctagga tccaaagcct 4740
   tgaagccacg gtagagaaac ttcttaccag cgagagtaag ctgaagcagg ctgcgctgac 4800
   cctggaggtg gagcgctccg ccctgctgca gatggtggag gagctgcgga ggcaaagcgc 4860
   ccggcccagc actccagagc cagactgcac ccagctggag cccacaggcg attgaccgct 4920
   gccagaagag actgtgcacc attaacactg tccaagcctt aatcaagaga gatggaagtc 4980
   agaggcagag aagagagaac ttctcaggga ggaaactggc tgaccagcct gcagatcctt 5040
   ttgagctcag aacttgggat tggaggacaa aagtctcaga gttattgttg tttttggttc 5100
   taatccgtcc cctttccagt cctggttgtt gtagctttag atggcatgga catgaataaa 5160
   ttacatttat ggttcctcct ccttcttgca gagacacagt gtgtggaaag agcattcatg 5220
   actgcagtgt tccaaaaaca agatgggtct gaaataattc ctcaggcaga agaaacgtga 5280
   ccctttgtca gtctgcaaga aaggttcctg ggagggtgcc cttgatgtca cctgaggtgc 5340
   agtgggccct tgtccatttt tattatggga cctaaggtga ccatgaggaa ggaagaaagg 5400
   ggtttattta aatcagatat attggttgtt cctgaatcca cggtcactcc cattggtact 5460
   gaagagagct catcttttac aaactcagtg ttttagtatg gtttttgctt accctttcaa 5520
   gtcacaggta aatgtttcat aaggaaatca cagtttttag attccctgtt attgctagtg 5580
   tgggacctgt agctgtgggg tctgacatat ttatttcttt ttgattgttt catatccaca 5640
   ctatagggtc acatatttat ttttgtctgt tgataaatga tgtaccactg ccacatcttc 5700
   ctttgagcct tgaacccctt ggaaatctga tttctcatag aaggaacaaa acataccaag 5760
   atttccaggg gctcctaaga tgctgaaaca ttgcaaaaga aaccagattg caatcaggtg 5820
   tatggcagct gacagctttt cagaagtagg aagtgtgttt tgaaagtact caaacacacg 5880
   cacgccttcc ttgggatggc gaggaccctg tgccttcaag cagagtgcag ctcacactca 5940
   ccctgcttct agctggtcct gtgtagagca tcacccatgt gtgcaacagc tttttttttt 6000
   cttttaacat acacacaaag gcttaggtta gggaagacca ccagccagga gccctggcta 6060
   tcggaattgg tcctgccttg ggctctgttg gccctcctgt tcctggaggt ctgctgcagc 6120
   gctgttggta ggaaaggagc cttgtaagtc tgggctgtaa ggagagacca tcaccgttgg 6180
   ggtcatttgg gatctcctag ccctatcaaa gggacactaa atggccagga gggatgtaag 6240
   actcccacat ggtcttgggt cagaggagag gtctctccat aaaccaacat gcccagtccc 6300
   agttggttgg agcaggggtt gtcagaggag ggtgacagtg gacagcttct ttccacattg 6360
   tattttatta tttgaatgtt ttaatgttga attttggaca tgttattact gttcaaaaaa 6420
   cactgactct acagtaagaa aagagcaata aaacagatta ttcac 6465
<210> 5
   <211> 30
   <212> DNA
   <213> Mus musculus
<400> 5
   tgcgtcgacg aagcagagac tgtggagtgg 30
<210> 6
   <211> 29
   <212> DNA
   <213> Mus musculus
<400> 6
   atcgtataca acaaccagga ctggaaagg 29
<210> 7
   <211> 19
   <212> DNA
   <213> Mus musculus
<400> 7
   agtggccact ccacagaag 19
<210> 8
   <211> 19
   <212> DNA
   <213> Mus musculus
<400> 8
   tcctgtactg ggccaaatg 19
<210> 9
   <211> 35
   <212> DNA
   <213> Mus musculus
<400> 9
   actccccgag cagatatgaa gattattccg agctg 35
<210> 10
   <211> 19
   <212> DNA
   <213> Mus musculus
<400> 10
   atgtggacca tcttcctgg 19
<210> 11
   <211> 19
   <212> DNA
   <213> Mus musculus
<400> 11
   gctctccact ggaattgtg 19
<210> 12
   <211> 27
   <212> DNA
   <213> Mus musculus
<400> 12
   ttcagctccg cctccacctc gtcttaa 27
<210> 13
   <211> 21
   <212> DNA
   <213> Mus musculus
<400> 13
   gagcaggtca gctgtcactt t 21
<210> 14
   <211> 20
   <212> DNA
   <213> Mus musculus
<400> 14
   tgagaccttg gcagtatcca 20
<210> 15
   <211> 25
   <212> DNA
   <213> Mus musculus
<400> 15
   aaaggttaaa tctggatctt gagtg 25
<210> 16
   <211> 22
   <212> DNA
   <213> Mus musculus
<400> 16 22
   ctcctcaagt ttgttttgct ca 22
<210> 17
   <211> 27
   <212> DNA
   <213> Mus musculus
<400> 17
   tataaagata atcagggctt aaactcg 27
<210> 18
   <211> 20
   <212> DNA
   <213> Mus musculus
<400> 18
   acctccacaa catgagcaca 20
<210> 19
   <211> 27
   <212> DNA
   <213> Mus musculus
<400> 19
   tttaagttgt gttaatgttg aaagttc 27
<210> 20
   <211> 20
   <212> DNA .
   <213> Mus musculus
<400> 20
   cacacatgca cgaacatgta 20
<210> 21
   <211> 19
   <212> DNA
   <213> Mus musculus
<400> 21
   gcatgtgcgt gtgcttatg 19
<210> 22
   <211> 24
   <212> DNA
   <213> Mus musculus
<400> 22
   aatgtatacc tccacaaaca cctc 24
<210> 23
   <211> 26
   <212> DNA
   <213> Mus musculus
<400> 23
   tagatgctgc ctaacaatta gtaagt 26
<210> 24
   <211> 24
   <212> DNA
   <213> Mus musculus
<400> 24
   tgggaatatt ttttcaagtg tctc 24
<210> 25
   <211> 20
   <212> DNA
   <213> Mus musculus
<400> 25
   agaattggga cgaagccttt 20
<210> 26
   <211> 25
   <212> DNA
   <213> Mus musculus
<400> 26
   tttgaaatca agattagcca ttacc 25
<210> 27
   <211> 25
   <212> DNA
   <213> Mus musculus
<400> 27
   aaaatcagtt aaggtagcac aatgc 25
<210> 28
   <211> 18
   <212> DNA
   <213> Mus musculus
<400> 28
   gttggccatc tttgctgg 18
<210> 29
   <211> 21
   <212> DNA
   <213> Mus musculus
<400> 29
   ccctgtgctc tggattagtt g 21
<210> 30
   <211> 20
   <212> DNA
   <213> Mus musculus
<400> 30
   tcaagaccag catcaaacca 20
<210> 31
   <211> 20
   <212> DNA
   <213> Mus musculus
<400> 31
   gtgcaggcct gaattgaaat 20
<210> 32
   <211> 19
   <212> DNA
   <213> Mus musculus
<400> 32
   caaagtgcct gtgcatgtg 19
<210> 33
   <211> 26
   <212> DNA
   <213> Mus musculus
<400> 33
   aagagagaca tacacaaaca gacaca 26
<210> 34
   <211> 27
   <212> DNA
   <213> Mus musculus
<400> 34
   tttcttaaca agtttctagc aatttcc 27
<210> 35
   <211> 27
   <212> DNA
   <213> Mus musculus
<400> 35
   tgtctgtctg tcattctgtc atatatg 27
<210> 36
   <211> 22
   <212> DNA
   <213> Mus musculus
<400> 36
   ccaaaaacca gatttgagat cc 22
<210> 37
   <211> 22
   <212> DNA
   <213> Mus musculus
<400> 37
   aagaccactt gaaaacagtg ca 22
<210> 38
   <211> 24
   <212> DNA
   <213> Mus musculus
<400> 38
   ggagatctaa atttcaaaac acca 24
<210> 39
   <211> 27
   <212> DNA
   <213> Mus musculus
<400> 39
   cattttaaaa cagaaatctg aagttcc 27
<210> 40
   <211> 22
   <212> DNA
   <213> Mus musculus
<400> 40
   gtgactaggt atgccaaatc cc 22
<210> 41
   <211> 27.
   <212> DNA
   <213> Mus musculus
<400> 41
   tcatgactat aaatgtattc atgtgtg 27
<210> 42
   <211> 23
   <212> DNA
   <213> Mus musculus
<400> 42
   cttcaaagac tcccatatac ccc 23
<210> 43
   <211> 21
   <212> DNA
   <213> Mus musculus
<400> 43
   cacagagaag aatctgcgag c 21
<210> 44
   <211> 26
   <212> DNA
   <213> Mus musculus
<400> 44
   ttgcaaagtg ttttaatcag tatttg 26
<210> 45
   <211> 26
   <212> DNA
   <213> Mus musculus
<400> 45
   ggttaagaaa aagacagaaa atctgc 26
<210> 46
   <211> 26
   <212> DNA
   <213> Mus musculus
<400> 46
   ggttaagaaa aagacagaaa atctgc 26
<210> 47
   <211> 19
   <212> DNA
   <213> Mus musculus
<400> 47
   acccctattc agtgccagg 19
<210> 48
   <211> 19
   <212> DNA
   <213> Mus musculus
<400> 48
   acccaggcca caaaagaac 19
<210> 49
   <211> 26
   <212> DNA
   <213> Mus musculus
<400> 49
   tcagtgataa tgacatggta gaagtg 26
<210> 50
   <211> 18
   <212> DNA
   <213> Mus musculus
<400> 50
   agagggtttc cttccccc 18
<210> 51
   <211> 18
   <212> DNA
   <213> Mus musculus
<400> 51
   tcaaaaccca gcctcctg 18
<210> 52
   <211> 23
   <212> DNA
   <213> Mus musculus
<400> 52
   agagtgtgac taacaggtga ggc 23
<210> 53
   <211> 20
   <212> DNA
   <213> Mus musculus
<400> 53
   ccatctctcc agcccctaga 20
<210> 54
   <211> 20
   <212> DNA
   <213> Mus musculus
<400> 54
   ctatgggcac cagtcagaca 20
<210> 55
   <211> 20
   <212> DNA
   <213> Mus musculus
<400> 55
   ggcaacacaa aatggactca 20
<210> 56
   <211> 20
   <212> DNA
   <213> Mus musculus
<400> 56
   ccaacatctc ctgtgtccct 20
<210> 57
   <211> 22
   <212> DNA
   <213> Mus musculus
<400> 57
   tcagtttgat tgggtttctt cc 22
<210> 58
   <211> 21
   <212> DNA
   <213> Mus musculus
<400> 58
   cacacataca tgcactcctg c 21
<210> 59
   <211> 21
   <212> DNA
   <213> Mus musculus
<400> 59
   accccatctc tgtctctctg a 21
<210> 60
   <211> 18
   <212> DNA
   <213> Mus musculus
<400> 60
   ctgggtgaga ggggaggt 18
<210> 61
   <211> 21
   <212> DNA
   <213> Mus musculus
<400> 61
   ttagcccact gtagcatcac c 21
<210> 62
   <211> 26
   <212> DNA
   <213> Mus musculus
<400> 62
   tatttttcac acaagaactt actggg 26
<210> 63
   <211> 21
   <212> DNA
   <213> Mus musculus
<400> 63
   tacctaccac aatgctctgg g 21
<210> 64
   <211> 20
   <212> DNA
   <213> Mus musculus
<400> 64
   tcccattgag ccttttagga 20
<210> 65
   <211> 27
   <212> DNA
   <213> Mus musculus
<400> 65
   ctgaaaaaaa aagttctcta ttcttga 27
<210> 66
   <211> 23
   <212> DNA
   <213> Mus musculus
<400> 66
   tgagcagaaa gtctattcca gaa 23
<210> 67
   <211> 22
   <212> DNA
   <213> Mus musculus
<400> 67
   cattttcatc ttattgccca tg 22
<210> 68
   <211> 24
   <212> DNA
   <213> Mus musculus
<400> 68
   acctaaagac agatacagga accc 24
<210> 69
   <211> 25
   <212> DNA
   <213> Mus musculus
<400> 69
   attatcagcc atcttcattc ataca 25
<210> 70
   <211> 27
   <212> DNA
   <213> Mus musculus
<400> 70
   gcctcacctt taaacaatac tactaga 27
<210> 71
   <211> 21
   <212> DNA
   <213> Mus musculus
<400> 71
   aagatgggaa aatcagggat g 21
<210> 72
   <211> 23
   <212> DNA
   <213> Mus musculus
<400> 72
   aaatgttccc ttcattttct tcc 23
<210> 73
   <211> 22
   <212> DNA
   <213> Mus musculus
<400> 73
   gcataaaatg gatttcctga gg 22
<210> 74
   <211> 20
   <212> DNA
   <213> Mus musculus
<400> 74
   tgtcccaaaa caaacaaccc 20
<210> 75
   <211> 20
   <212> DNA
   <213> Mus musculus
<400> 75
   aagccagaag cctgttgtgt 20
<210> 76
   <211> 23
   <212> DNA
   <213> Mus musculus
<400> 76
   gtctgttgtg agttcagtag ggg 23
<210> 77
   <211> 20
   <212> DNA
   <213> Mus musculus
<400> 77
   ccatgcccag tctggtatct 20
<210> 78
   <211> 24
   <212> DNA
   <213> Mus musculus
<400> 78
   ttttactttc cactaagcat tccc 24
<210> 79
   <211> 21
   <212> DNA
   <213> Mus musculus
<400> 79
   ccttccacat acataggcag g 21
<210> 80
   <211> 20
   <212> DNA
   <213> Mus musculus
<400> 80
   cctcagtgct ggatggattt 20
<210> 81
   <211> 20
   <212> DNA
   <213> Mus musculus
<400> 81
   tacacaggga aaggacaggg 20
<210> 82
   <211> 22
   <212> DNA
   <213> Mus musculus
<400> 82
   agggagattt tggattagag gc 22
<210> 83
   <211> 20
   <212> DNA
   <213> Mus musculus
<400> 83
   gaggactcct tcgatttccc 20
<210> 84
   <211> 21
   <212> DNA
   <213> Mus musculus
<400> 84
   ttcctaagca ggaactgacc a 21
<210> 85
   <211> 24
   <212> DNA
   <213> Mus musculus
<400> 85
   acttcttgtt actgcatgtg aagc 24
<210> 86
   <211> 22
   <212> DNA
   <213> Mus musculus
<400> 86
   caagagagca gagttagtgg ca 22
<210> 87
   <211> 25
   <212> DNA
   <213> Mus musculus
<400> 87
   agtggcataa cttcattcaa taatg 25
<210> 88
   <211> 27
   <212> DNA
   <213> Mus musculus
<400> 88
   ggaatcaagt tgttttttaa atttacc 27
<210> 89
   <211> 20
   <212> DNA
   <213> Mus musculus
<400> 89
   agggcagcag caacaaatac 20
<210> 90
   <211> 27
   <212> DNA
   <213> Mus musculus
<400> 90
   tcatcgtata aatgtatcaa ttctcaa 27
<210> 91
   <211> 25
   <212> DNA
   <213> Mus musculus
<400> 91
   agcttttact gaaatgtttt gtagg 25
<210> 92
   <211> 20
   <212> DNA
   <213> Mus musculus
<400> 92
   tgagcaggag cctatttgtg 20
<210> 93
   <211> 20
   <212> DNA
   <213> Mus musculus
<400> 93
   cccttcagag ccaagagaaa 20
<210> 94
   <211> 20
   <212> DNA
   <213> Mus musculus
<400> 94
   ttcctttgcc tctgtcacct 20
<210> 95
   <211> 20
   <212> DNA
   <213> Mus musculus
<400> 95
   cacgcctcca ttagaacctc 20
<210> 96
   <211> 22
   <212> DNA
   <213> Mus musculus
<400> 96
   tttcatggat ctcttaggca ca 22
<210> 97
   <211> 20
   <212> DNA
   <213> Mus musculus
<400> 97
   cgctgaattc ttcccaagtg 20
<210> 98
   <211> 22
   <212> DNA
   <213> Mus musculus
<400> 98
   ggtcatctct aggcaggatg tt 22
<210> 99
   <211> 22
   <212> DNA
   <213> Mus musculus
<400> 99
   ggtcaagtga aatcttcctg ct 22
<210> 100
   <211> 21
   <212> DNA
   <213> Mus musculus
<400> 100
   ccagttaagc atttccccct a 21
<210> 101
   <211> 20
   <212> DNA
   <213> Mus musculus
<400> 101
   gccctgcaga tagacgcttt 20
<210> 102
   <211> 20
   <212> DNA
   <213> Mus musculus
<400> 102
   acccaaggtt ttctggctct 20

## Description

The present invention relates to the gene TBC1D1 and its use as a genetic marker in the response to dietary fat, and obesity and diabetes. TBC1D1 provides a valuable tool both for diagnostic as well as therapeutic approaches, in order to treat or prevent obesity or diabetes, in particular in response to dietary fat.

### Description

The incidence of obesity has reached epidemic levels in the developed world (Hedley, A.A., Ogden, C.L., Johnson, C.L., Carroll, M.D., Curtin, L.R. and Flegal, K.M. (2004) Prevalence of overweight and obesity among US children, adolescents, and adults, 1999-2002. JAMA, 291, 2847-2850, Flegal, K.M., Carroll, M.D., Ogden, C.L. and Johnson, C.L. (2002) Prevalence and trends in obesity among US adults, 1999-2000. JAMA, 288, 1723-1727). If unchecked, the rising rates of obesity (and its associated morbidities) pose a significant threat to human health and a costly burden to public healthcare systems. Unfortunately, patients with severe obesity [usually defined as having a body-mass index (BMI) of over 35] have very few medical options. In fact, with the exception of surgical intervention, there are no effective long-term therapies (Brolin, R.E. (2002) Bariatric surgery and long-term control of morbid obesity. JAMA, 288, 2793-2796). A general goal is to define the genetic etiology of obesity predisposition to enable the development of effective therapeutic interventions.

Obesity is a complex genetic disease. Although it is clearly heritable (Price, R.A., Ness, R. and Laskarzewski, P. (1990) Common major gene inheritance of extreme overweight. Hum. Biol., 62, 747-765; Hunt, S.C., Hasstedt, S.J., Kuida, H., Stults, B.M., Hopkins, P.H. and Williams, R.R. (1989) Genetic heritability and common environmental components of resting and stressed blood pressures, lipids, and body mass index in Utah pedigrees and twins. Am. J. Epidemiol., 129, 625-638. Bell, C.G., Walley, A.J. and Froguel, P. (2005) The genetics of human obesity. Nat. Rev. Genet., 6, 221-234), e.g., first-degree relatives of obese patients have up to a 9-fold increase in disease risk (Price, R.A. and Lee, J.H. (2001) Risk ratios for obesity in families of obese African-American and Caucasian women. Hum. Hered., 51, 35-40), the segregation of the disease in obesity-prone families is non-Mendelian (Borecki, I.B., Higgins, M., Schreiner, P.J., Arnett, D.K., Mayer-Davis, E., Hunt, S.C. and Province, M.A. (1998) Evidence for multiple determinants of the body mass index: the National Heart, Lung, and Blood Institute Family Heart Study. Obes. Res., 6, 107-114). Also, many studies have provided evidence to suggest that risk for obesity is determined not only by specific genotypes but also by significant gene/gene and gene/environment interactions (Dong, C., Wang, S., Li, W.D., Li, D., Zhao, H. and Price, R.A. (2003) Interacting genetic loci on chromosomes 20 and 10 influence extreme human obesity. Am. J. Hum. Genet., 72, 115-124; Dong, C., Li, W.D., Li, D. and Price, R.A. (2005) Interaction between obesity-susceptibility loci in chromosome regions 2p25-p24 and 13q13-q21. Eur. J. Hum. Genet., 13, 102-108; Warden, C.H., Yi, N. and Fisler, J. (2004) Epistasis among genes is a universal phenomenon in obesity: evidence from rodent models. Nutrition, 20, 74-77; Lewis, C.E., North, K.E., Arnett, D., Borecki, LB., Coon, H., Ellison, R.C., Hunt, S.C., Oberman, A., Rich, S.S. and Province, M.A.(2005) Sex-specific findings from a genome-wide linkage analysis of human fatness in non-Hispanic whites and African Americans: the HyperGEN study. Int. J. Obes. Relat. Metab. Disord., 29, 639-649).

There have been many attempts to identify genes involved in the predisposition to common obesity, but presumably because of the genetic complexity, these efforts have had only mixed success (Bell, C.G., Walley, A.J. and Froguel, P. (2005) The genetics of human obesity. Nat. Rev. Genet., 6, 221-234., Pérusse, L., Rankinen, T., Zuberi, A., Chagnon, Y.C., Weisnagel, S.J., Argyropoulos, G., Walts, B., Snyder, E.E. and Bouchard, C. (2005) The human obesity gene map: the 2004 update. Obes. Res., 13, 381-490). Studies focused on rare forms of obesity have been more productive. Mutations in several genes have been linked to monogenic obesity. Also, several genes have been implicated in syndromic obesity (i.e. obesity with other clinically significant phenotypes not usually associated with the disease). However, genetic causes of the common form of this disease remain poorly defined.

Dietary fat intake is considered a major factor in the development of obesity and insulin resistance. In mice, sensitivity to dietary fat is strain specific, and consequently dependent on a genetic basis (West, D.B., Boozer, C.N., Moody, D.L. and Atkinson, R.L. Dietary obesity in nine inbred mouse strains. Am J Physiol 262, R1025-32 (1992). Wuschke, S., Dahm, S., Schmidt, C., Joost, H.G. and Al-Hasani, H. A meta-analysis of quantitative trait loci associated with body weight and adiposity in mice. Int J Obes Lond) (2006) Rankinen, T. et al. The human obesity gene map: the 2005 update. Obesity (Silver Spring) 14, 529-644 (2006)). Weight gain in response to dietary fat/caloric intake is dependent on the genetic composition of an individual. However, the genes and variants that determine the sensitivity to dietary fat are largely unknown. Since obesity is a major risk factor for the development of type-2 diabetes mellitus, cardiovascular diseases, and perhaps certain types of cancer, it is imperative to identify genetic risk factors for diet-induced adiposity and weight gain. Moreover, it is well established that obesity coincides with reduced insulin sensitivity and promotes impaired glucose tolerance. Nevertheless, it is still debated how increased fat mass affects insulin action on the molecular level.

The sequence of *Homo sapiens* TBC1 (tre-2/USP6, BUB2, cdc16) domain family, member 1 (TBC1D1) is described in the database under Accession number NM_015173. TBC1D1 is described as a founding member of a family of proteins sharing a 180- to 200-amino acid TBC domain presumed to have a role in regulating cell growth and differentiation. These proteins share significant homology with TRE2 (USP6; MIM 604334), yeast Bub2, and CDC16 (MIM 603461)

WO 00/08209 concerns genomic and cDNA sequences of the human TBC-1 gene and polypeptides encoded by the TBC-1 gene. WO 00/08209 also deals with antibodies directed specifically against such polypeptides that are useful as diagnostic reagents in *prostate cancer.* WO 00/08209 further encompasses biallelic markers of the TBC-1 gene useful in genetic analysis.

The US patent application US 2003/0096782 A2 describes TCB1D1 (referred to as KIAA1108 therein) to be involved in cardiac disease. WO 02/099049 A2 discloses that TCB1D1 is a modulator of the p53 pathway.

Stone et al. (TBC1D1 is a candidate for a severe obesity gene and evidence for a gene/gene interaction in obesity predisposition. Hum Mol Genet. 2006; 15(18):2709-20) describe a genetic variation in the human gene TBC1D1 that confers risk for severe obesity in females. They identified a coding variant (R125W) in TBC1D1 that segregated with the disease in 4p15-14-linked obesity pedigrees. They postulate that R125W affects obesity susceptibility, delimits the location of an obesity gene at 4q34-35, and mention that the protein is structurally similar to a known regulator of insulin-mediated Glut4 translocation (AS 160).

Stone et al. further describe that a) R125W confers risk for obesity only in the context of a specific gene/gene interaction, b) the W allele is not enriched in random severely obese cases, and c) that TBC1D1 confers risk for severe obesity in females. Stone et al. do not describe d) deletions or truncations of TBC1D1, e) a lack of functionality of TBC1D1 in obese cases, and f) a link of TBC1D1 with the diet of the obese cases.

In view of the above, it is an object of the present invention to provide novel genetic markers that are involved in the response to dietary fat, and obesity. These markers provide valuable tools, both for diagnostic as well as therapeutic approaches in order to treat or prevent obesity.

According to a first aspect thereof, the object of the present invention is solved by providing a method for diagnosing obesity or diabetes or an increased risk for obesity or diabetes in a mammal, comprising analysis of the biological activity and/or expression of TBC1D1 in a biological sample obtained from said mammal to be diagnosed, wherein a loss of function mutation in the Rab-specific GTP-ase activating TBC domain in the gene for TBC1D1 or a lack or reduced biological activity of TBC1D1 in said mammal is indicative for an absence or a reduced risk for obesity or diabetes in said mammal, wherein said biological activity of TBC1D1 is the activity of the Rab-specific GTP-ase activating TBC domain.

According to another aspect thereof, the object of the present invention is solved by providing the use of a cell that is transformed with an expression vector comprising a polynucleotide encoding a polypeptide selected from a) a polypeptide that is encoded by SEQ ID NO: 3 or 4 (providing the human and mouse mRNA sequences, respectively), b) a polypeptide having an amino acid sequence having a 90% or more homology with an amino acid sequence of SEQ ID NO: 1 or 2 (providing the human and mouse amino acid sequences, respectively), and exhibiting a TBC1D1 activity, c) a polypeptide having an amino acid sequence in which 1 to 10 amino acids are deleted, substituted, and/or added in the amino acid sequence according to SEQ ID NO: 1 or 2, and exhibiting a TBC1D1 activity, and d) a polypeptide consisting of an amino acid sequence of SEQ ID NO: 1 or 2, wherein said cell expresses said polypeptide, or a non-human transgenic mammal overexpressing TBC1D1 and/or carrying a TBC1D1 genetic reporter-construct, wherein said biological activity of TBC1D1 is the activity of the Rab-specific GTP-ase activating TBC domain as a screening tool in a method for screening for an agent for treating or preventing obesity or diabetes according to the present invention.

According to yet another aspect thereof, the object of the present invention is solved by providing a method of identifying a compound that modulate the expression and/or biological activity of TBC1D1 in a cell, comprising the steps of a) contacting a cell expressing TBC1D1 with at least one potentially interacting compound, and b) measuring the modulation of the expression and/or biological activity of TBC1D1 in said cell, wherein said biological activity of TBC1D1 is the activity of the Rab-specific GTP-ase activating TBC domain. Preferred is a method according to the present invention, wherein said compound as identified is an inhibitor of the expression and/or biological activity of TBC1D1 in said cell.

Described is furthermore a pharmaceutical composition for treating or preventing obesity or diabetes, which includes a compound that modulate the expression and/or biological activity of TBC1D1 in a cell obtainable by a method according to the present invention.

Also described is the use of a modulator of the expression and/or the biological activity of TBC1D1 in a cell for the manufacture of a pharmaceutical composition for treating or preventing obesity or diabetes. Preferred is a method according to the present invention, wherein said obesity or diabetes is dietary fat-related. Further described is a use, wherein said modulator is an inhibitor of the expression and/or biological activity of TBC1D1.

The present invention is technically based on genome wide scans of outcross populations of the New Zealand Obese (NZO) mouse and the lean Swiss Jim Lambert (SJL) strain, whereby the inventors have previously identified a major QTL for high fat diet (HFD) induced obesity *(Nob1;* LOD score 7.9) on chromosome 5. Additional crossbreeding experiments indicated that *Nob1* represents an obesity suppressor from the SJL strain.

In a combined approach of gene expression analysis and sequencing, the inventors identified a SJL specific 7 bp deletion in the *Tbc1d1* gene (4048delACTCGCT) which results in a truncated protein lacking the TBC/Rab GTPase activating (GAP) domain. Tbc1d1 was identified by genetic and molecular analyses of mouse models that are inherently sensitive or resistant to diet-induced obesity and diabetes, respectively. The data of the inventors indicated that the integrity of the Tbc1d1 gene product is required for diet-induced weight gain. In fact, Tbc1d1 activity (presumably through its GTPase activating protein (GAP) domain) appears to determine the capacity of gaining weight in response to a given caloric input: According to the data, animals with a high activity/high amount of intact Tbc1d1 gene product gain more weight than animals with low activity/low amount of intact Tbc1d1 in response to a caloric challenge, such as high fat diet induced obesity. This phenomenon is designated herein as "dietary fat-related", although the caloric challenge is not exclusively limited to calories derived from dietary fat.

Tbc1d1 is a member of a larger family of putative signalling proteins that are highly conserved in mammals. Mouse and human Tbc1d1 proteins are 80-90 % identical, and the tissue expression profile of Tbc1d1 appears to be identical in both species. Tbcld4 also known as AS 160 is a critical protein for insulin signalling, and highly homologous to Tbc1d1. It is established that Tbcld4 exerts the same crucial function in insulin signalling in both species, human and mouse. Thus, the biological function of Tbc1d1 is highly likely to be conserved between species. As a matter of fact, a previous study from the Utah group (Stone et al., see above) has reported genetic evidence of linkage of the Tbc1d1 locus to obesity in humans.

Introgression of the *Nob1* segment of the SJL chromosome 5 into a mixed NZO/C57BL/6J background markedly reduces body weight and blood glucose levels. Tbc1d1 is related with the insulin signalling protein Tbcld4 (AS160), and is highly expressed in skeletal muscle, heart, pancreas, kidney and hypothalamus; its expression in skeletal muscle is increased by high fat diet. The data shows that deletion of *Tbc1d1* suppresses high fat diet induced obesity caused by the polygenic NZO background. Thus, *Tbc1d1* is involved in a novel pathway that regulates lipid metabolism and energy homeostasis in skeletal muscle.

In view of this, possible applications of the present invention for humans include:
a) Diagnostic approaches: Since Tbc1d1 activity determines the susceptibility for (high fat) diet-induced weight gain, genetic tests can be developed to assess an individual risk for developing overweight/obesity and or diabetes, and to develop a personalized diet and/or treatment as an intervention approach (see above). These tests are also envisaged to be applicable for diagnosing/treating weight disorders in general, i.e. also weight loss as a result of e.g. eating disorders or during cancer therapy.
b) Pharmaceutical (obesity/diabetes) and therapeutic approaches: The inventors' data indicate that an inhibitor of Tbc1d1 action can be used as an obesity drug in mammals/humans. Moreover, since reduced levels of Tbc1d1 also result in reduced blood glucose levels in a mouse model for the metabolic syndrome, a Tbc1d1 inhibitor is very likely to be used as a diabetes drug as well, and also for treating other components of the metabolic syndrome, including hyperlipidemia, hypercholesterinemia, high blood pressure, and aspects of vascular and cardiovascular diseases that are usually secondary to insulin resistance and diabetes. Since the lean SJL mice that are deficient in Tbc1d1 appear to be healthy, normoglycemic and without major metabolic derangements, a pharmacological inhibitor of Tbc1d1 is likely to have a high therapeutic index, i.e. appears to be tolerable and safe.

Furthermore, as mentioned, a high fat diet is known to induce insulin resistance, obesity and diabetes in certain individuals. The majority of individuals with type-2 diabetes are obese. However, only a minor fraction of obese individuals develop type-2 diabetes. Thus, certain variants of Tbc1d1 that might interact with the environment (e.g. dietary fat) could provide a link between diabetes and obesity. Tbc1d1 is highly expressed in skeletal muscle, the main site of glucose disposal and an important mediator or energy homeostasis. The inventors' data show that Tbc1d1 is phosphorylated at a specific threonine by AKT2 kinase in response to insulin. In cohort studies with human individuals, the inventors were able to show an association between TBC1D1 variants and obesity and diabetes.

Moreover, Tbc1d1 defines a novel pathway with valuable downstream targets that regulate lipid metabolism, energy homeostasis and/or other, yet unknown biological entities. In particular, the inventors could show that Tbc1d1 modulates fatty acid uptake and oxidation in cultured muscle cells.

Finally, the ortholog of the marker tbc1d1 is of use in order to identify animals that gain more weight in response to a caloric, such as a high fat-containing, diet. The marker thus can be used for breeding purposes of cattle by, for example, selecting those animals that do not have a mutated tbc1d1 marker.

The term "homology" as used herein means a value obtained by a BLAST [Basic local align-ment search tool; Altschul, S. F. et al., J. Mol. Biol., 215, 403-410, (1990)] search. The homology in the amino acid sequence may be calculated by a BLAST search algorithm. More particularly, it may be calculated using a bl2seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

Preferred is a diagnostic method according to the present invention, wherein said obesity or diabetes is dietary fat-related. As a major finding in the context of the present invention, animals with a high activity/high amount of intact Tbc1d1 gene product gain more weight than animals with low activity/low amount of intact Tbc1d1 in response to a caloric challenge, such as high fat diet. Thus, the animals with a high activity/high amount of intact Tbc1d1 gene product do not always become obese, but gain more weight in response to a caloric challenge, such as a high fat-containing diet.

Preferred is a diagnostic method according to the present invention, wherein said mammal to be diagnosed is a mouse, rat or human.

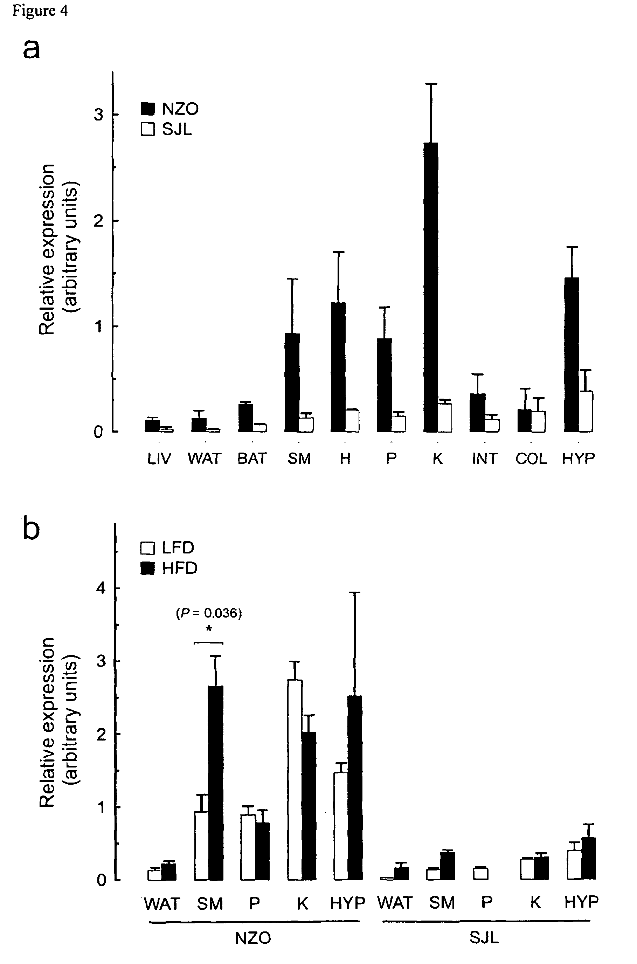

Preferred is a diagnostic method according to the present invention, wherein said biological sample contains biological material that is derived from skeletal muscle, heart, pancreas, kidney, and/or hypothalamus of said mammal to be diagnosed. In C57BL/6J and NZO mice, *Tbc1d1* is expressed mainly in skeletal muscle, heart, pancreas, kidney and hypothalamus, and only low levels of expression were detected in white and brown adipose tissue, liver, intestine, and colon (see Figure 4a).

Both the expression and/or abundance of TBC1D1 or the mRNA thereof as well as the biological activity can be determined with any suitable assay known to the person of skill. Common assays involve PCR, Northern-, Western- or Southern-Blots, and/or enzymatic assays. Thus, preferred is a method according to the present invention, wherein said diagnosis comprises the use of TBC1D1-specific antibodies, hybridisation analysis, quantitative PCR, mRNA analysis, quantitative analysis of the amount of TBC1D1-protein, sequencing of the TBC1D1-locus, and/or an analysis of the activity of TBC1D1 in said sample.

Further preferred is a method according to the present invention, wherein TBC1D1 is down-regulated at least 3-fold in skeletal muscle. As found during the experiments in the context of the present invention, in skeletal muscle of C57BL/6J and NZO mice, expression of *Tbc1d1* is significantly increased ∼3-fold in response to high fat diet, whereas no significant changes were observed in the other tissues (Figure 4b). As a result, in skeletal muscle from animals raised on a high-fat diet, the levels of *Tbc1d1* mRNA are increased by about an order of magnitude in NZO mice compared to SJL mice, respectively.

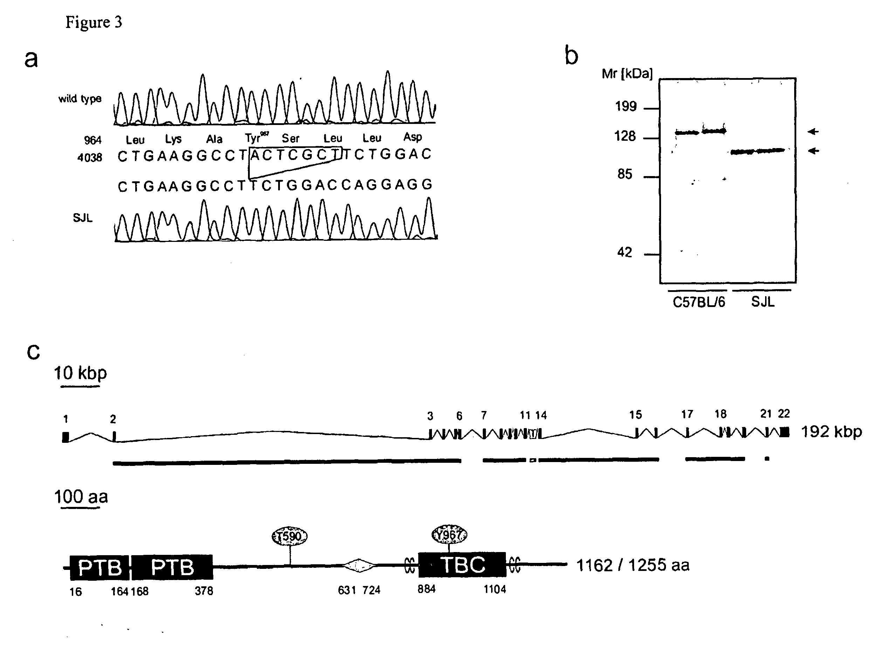

In the method according to the present invention, the gene for TBC1D1 contains a loss-of function deletion, more particularly a 7 bp deletion in exon 18 of the mouse gene for TBC1D1 or the homologous deletion in the human gene for TBC1D1, corresponding to A4048-T4054 of SEQ ID No. 3. As identified in the in vitro translation studies in the context of the present invention (Figure 3), the deletion represents a loss-of function (LOF) mutation since the mutated protein lacks most invariant residues of the GTPase-activating TBC domain, including the conserved and catalytically essential glutamine finger (YxQ978) (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)).

Described is also a screening tool for an agent for treating or preventing obesity or diabetes, wherein said tool is preferably selected from a polypeptide consisting of an amino acid sequence of SEQ ID NO: 1 or 2 or an mammalian ortholog of these polypeptides (by definition, orthologs are genes that are related by vertical descent from a common ancestor and encode proteins with the same function in different species).

In another aspect of the present invention, the screening tool for an agent for treating or preventing obesity or diabetes is a cell which is transformed with an expression vector comprising a polynucleotide encoding a polypeptide according to SEQ ID NO: 1 or 2 or an mammalian ortholog of these polypeptides, and expresses said polypeptide. The cell can be a prokaryotic or eukaryotic cell, and the expression constructs can be present extrachromosomally or integrated into the chromosome. The polypeptide can be expressed in the form of a fusion protein, for example together with an enzymatically active moiety as reporter-construct, in order to be able to detect the expression product. Preferred host cells are derived from cells selected from the skeletal muscle, heart, pancreas, kidney, and/or hypothalamus.

In another aspect of the present invention, the screening tool for an agent for treating or preventing obesity or diabetes is a non-human transgenic mammal overexpressing TBC1D1 and/or carrying a TBC1D1 genetic reporter-construct. Preferred is a transgenic mouse, rat, pig, goat or sheep, wherein the reporter-construct is preferably expressed in cells selected from the skeletal muscle, heart, pancreas, kidney, and/or hypothalamus of said animal. Methods to produce these non-human transgenic mammal overexpressing TBC1D1 and/or carrying a TBC1D1 genetic reporter-construct are well known to the person of skill in the art.

As already mentioned above, according to yet another aspect thereof, the object of the present invention is solved by providing a method of identifying a compound that modulate the expression and/or biological activity of TBC1D1 in a cell, comprising the steps of a) contacting a cell expressing TBC1D1 with at least one potentially interacting compound, and b) measuring the modulation of the expression and/or biological activity of TBC1D1 in said cell. Preferred is a method according to the present invention, wherein said compound as identified is an inhibitor of the expression and/or biological activity of TBC1D1 in said cell.

This method is suitable for the determination of compounds that can interact with TBC1D1 and to identify, for example, inhibitors, activators, competitors or modulators of TBC1D1, in particular inhibitors, activators, competitors or modulators of the enzymatic activity of TBC1D1. Also described are compounds that modulate the expression of TBC1D1 in a cell/in cells.

Such a modulator can be an antisense molecule or siRNA molecule that binds to the mRNA transcribed from the Tbd1d1 gene and inhibits its translation into a functional protein. In particular, such siRNA molecules preferably have a length of 18-22 nucleotides and are reverse complementary in sequence to the Tbd1d1 mRNA sequence. A suited siRNA molecule can be determined by a skilled artisan based on the information given herein together with his general knowledge.

Such a modulator can be transfected into a cell using standard cell culture techniques. E.g., lipofection, gene gun bombardment, electroporation, and/or virus infection (e.g. using lentiviruses) can be used.

The term "contacting" in the present invention means any interaction between the potentially binding substance(s) with TBC1D1, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and TBC1D1 (or a functional part thereof) is subsequently contacted with such a chip.

The TBC1D1 employed in a method of the present invention can be a full length protein or a fragment with N/C-terminal and/or internal deletions. Preferably the fragment is either an N-terminal fragment comprising the enzymatic region of the polypeptide or a C-terminal fragment comprising the cytoplasmic region, depending on whether potentially interacting compounds are sought that specifically interact with the N- or C-terminal fragment.

The potentially binding substance, whose binding to TBC1D1 is to be measured, can be any chemical substance or any mixture thereof. For example, it can be a substance of a peptide library, a combinatory library, a cell extract, in particular a plant cell extract, a "small molecular drug", a protein and/or a protein fragment.

Measuring of binding of the compound to TBC1D1 can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either TBC1D1 or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to TBC1D1 or TBC1D1 fragments. The effect of the binding of the compound or the activity of TBC1D1 can also be measured indirectly, for example, by assaying an enzymatic activity of TBC1D1 after binding, such as the phosphorylation at a specific threonine by AKT2 kinase in response to insulin, and the like.

As a further step after measuring the binding of a potentially interacting compound and after having measured at least two different potentially interacting compounds at least one compound can be selected, for example, on grounds of the measured binding activity or on grounds of the detected increase or decrease of TBC1D1 activity and/or expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with TBC1D1 and subsequently binding of the modified compounds to the TBC1D1 polypeptide is measured. In this step, both the binding per se can be measured and/or the effect of the function of the TBC1D1 like, e.g. the enzymatic activity of the polypeptide can be measured. If needed the steps of selecting the binding compound, modifying the binding compound, contacting the binding compound with a TBC1D1 polypeptide and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity of the TBC1D1 polypeptide(s).

Also described is a method where the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills.

Similar to the strategies for identifying compounds that interact with TBC1D1, compounds can be identified that modulate the expression of TBC1D1 in a cell. In preferred strategies, the expression of TBC1D1 can be monitored using a reporter-construct for TBC1D1 (in order to analyse the translation efficiency and/or stability of the TBC1D1 polypeptide), for an example a fusion protein comprising a detectable fusion member (such as an enzymatic or fluorophoric group), or the amount of mRNA as present in a cell can be measured, for example, by Northern blot. The expression can also be analysed and monitored by using chip-analysis or rtPCR. Preferred compounds that modulate the expression of TBC1D1 in a cell are selected from specific antisense oligonucleotide(s), or siRNA(s) for RNAi. Respective methods are well described in the literature and known to the person of skill.

Described is also a method for manufacturing a pharmaceutical composition for treating or preventing obesity or diabetes, comprising the steps of: performing a screening method according to the present invention, and formulating said modulator as screened and identified into a pharmaceutical composition.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used.

The present invention also describes a pharmaceutical composition for treating or preventing obesity or diabetes, obtainable by a method according to the method as above.

In certain embodiments, the compound (modulator) is administered to the subject by administering a recombinant nucleic acid. Preferably, the recombinant nucleic acid is a gene therapy vector.

Described is a method or use as above, wherein the pharmaceutical composition further comprises additional pharmaceutically active ingredients that modulate the diseases to be treated, such as obesity and diabetes.

Also described is a method for treating or preventing obesity or diabetes, comprising a diagnostic method according to the present invention as above, and providing a treatment to said mammal based on, at least in part, the result of said diagnosis. In general, the attending physician will base a treatment, and in particular obesity- and/or diabetes-preventive measures, on the diagnostic data regarding TBC1D1 of said patient, wherein said patient diagnostic data regarding TBC1D1 can be integrated with other individual patient data (clinical data, family history, DNA, etc.), and a treatment can also be performed based on the combination of these factors. This method for example involves integrating individual diagnostic data with patient clinical information and general healthcare statistics to enable, for example, the application of personalized medicine to the mammal. Significant information about drug effectiveness, drug interactions, physiologic heart regularities and other patient status conditions could be correlated with the specific diagnostic data.

Also described is a method of treating or preventing obesity or diabetes in a mammal, comprising administering to said mammal an effective amount of a pharmaceutical composition as above. Preferably, an inhibiting active agent is administered in form of a pharmaceutical composition, such as an antibody, antisense nucleotide or an inhibiting binding compound. Preferably, said patient is a human being. Treating is meant to include, e.g., preventing, treating, reducing the symptoms of, or curing the disease or condition.

Another aspect then relates to a monoclonal antibody recognizing an epitope derived from the N-terminus of mouse Tbc1d1 (Genbank NP_062610) amino acids 215-229: CTGQPSAPGPRPMRKS-CONH2 (Cys1 is used for coupling) or an ortholog of this peptide sequence.

An "effective amount" is an amount of the compound(s) as mentioned above that a) acts on the expression and/or abundance of TBC1D1 as analysed, and which alleviates symptoms as found for obesity (which might overlap with diabetic symptoms in general). Alleviating is meant to include, e.g., preventing, treating, reducing the symptoms of, or curing the disease or condition.

The specification also describes a method for treating a subject at risk for obesity and/or diabetes, in particular dietary fat-related, wherein a therapeutically effective amount of a modulator as above is provided. Being at risk for the disease can result from, e.g., a family history of the disease, a genotype which predisposes to the disease, or phenotypic symptoms which predispose to the disease. A further aspect is the use of a modulator of the expression and/or the biological activity of TBC1D1 in a cell for the manufacture of a phar maceutical composition for treating or preventing obesity or diabetes. Preferred is a use, wherein said obesity or diabetes is dietary fat-related (as described above). More preferably, said modulator is an inhibitor of the expression and/or biological activity of TBC1D1.

The following figures, sequences, and examples merely serve to illustrate the invention and should not be construed to restrict the scope of the invention to the particular embodiments of the invention described in the examples.

SEQ ID NOs 1 and 2 show the polypeptides TBC1D1 that are encoded by SEQ ID NO: 3 or 4, respectively.

SEQ ID NOs 3 and 4 show the nucleotide sequence of the mouse gene for TBC1D1 (Genbank Acc No. AK122445) and the human gene for TBC1D1 (Genbank Acc No. NM_015173), respectively.

SEQ ID NOs 5 to 102 show primers and probes as used in the examples below (see also table 1).

### Figures

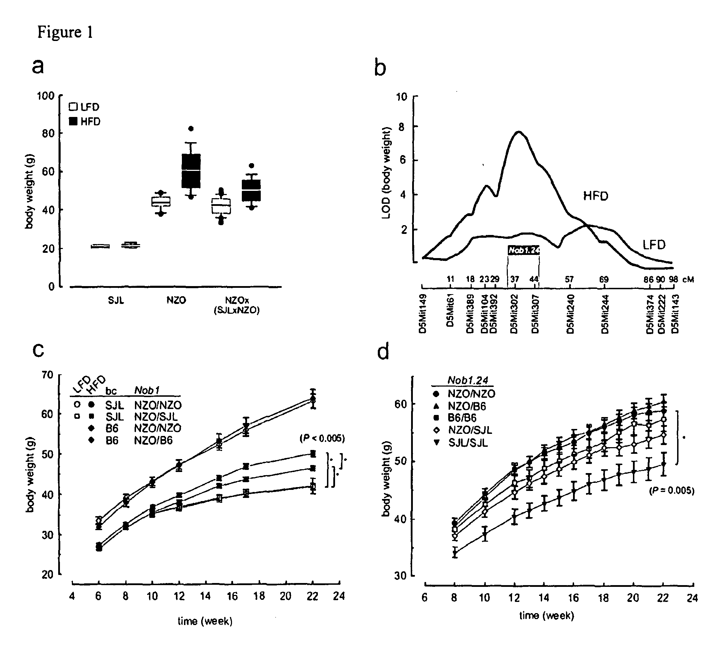

**Figure 1** shows that the adiposity QTL *Nob1* constitutes a diet-dependent obesity suppressor from the lean SJL strain. (a) boxplot representation of the body weight distribution at week 22 of SJL mice, NZO mice, and a NZO × F1(SJL×NZO) backcross population raised on a low-fat (LFD) or high-fat diet (HFD), respectively. (b) LOD ratios on chromosome 5 for body weight at week 22 in the combined NZO × F1(SJL×NZO) backcross population on a LFD (n= 297) or HFD (n= 585). Body weights were adjusted for gender differences. *Nob1*.24 represents the critical 24 Mb region of the *Nob1*-QTL. (**c**) *Nob1* genotype- and diet-dependent body weight (± s.e.m) of female mice from two different backcross populations of NZO and SJL (SJL bc; n= 17 - 89), and NZO and C57BL/6J (B6 bc; n= 25-34). (**d**) genotype-dependent body weight (± s.e.m) of female F2 mice from a cross of RCS B6.SJL-*Nob1.24* and NZO raised on a HFD (n= 19 - 58).

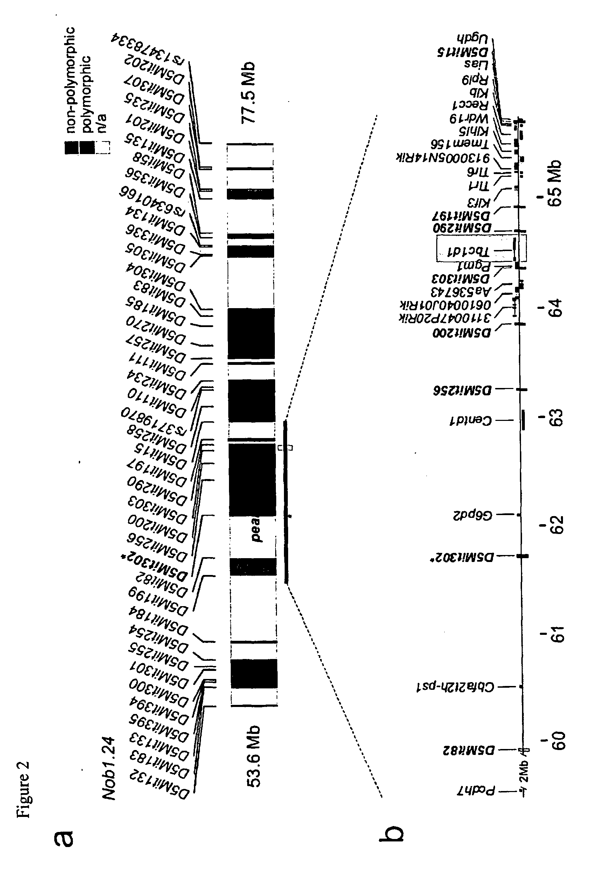

**Figure 2** shows fine mapping of the critical region of *Nob1.* (**a**) The chromosomal segment from SJL in RCS B6.SJL*-Nob1.24* mice was used for pairwise comparison of microsatellite fragment length polymorphisms and SNPs between parental NZO and SJL mice. The markers define polymorphic (red) and non-polymorphic regions (green), and regions residing between adjacent blocks typed as NZO or SJL (white). (**b**) Physical gene map of the *Nob1* peak region.
**Figure 3** shows a SJL-specific deletion in exon 18 of *Tbc1d1* which results in premature termination of the protein and deletion of the TBC domain. (**a**) Electropherograms of the wild type (C57BL/6J) and mutant (SJL) alleles of *Tbc1d1* showing sequences around the sites of the deletion (del(A4048-T4054)). The frameshift mutation results in termination of the protein at TGA⁴¹²⁹. (**b**) Autoradiograph of *in vitro* translated [³⁵S]*Tbc1d1* (arrows) from C57BL/6J (app. Mᵣ∼141 kDa) and SJL (app. Mᵣ∼117 kDa) after 10% SDS-PAGE. (c) Gene structure of *Tbc1d1* and proposed domain architecture of the protein. Indicated is the conserved phosphorylation site for AKT (T⁵⁹⁰) and site of the mutation (Y⁹⁶⁷) in the protein. PTB, phosphotyrosine binding domain; TBC, tre-2/USP6, BUB2, cdc16 domain; CC, coiled-coil domain; blue diamond, 93 amino acid insertion by alternative splicing of exons 12 and 13.
**Figure 4** shows the tissue distribution and diet-induced expression of *Tbc1d1.* (**a**) Relative expression levels of *Tbc1d1* in different tissues. (**b**) Expression levels in tissues from LFD and HFD fed mice. Quantification of gene expression was performed with samples from 8 wk old mice (n = 6) by TaqMan-Realtime-PCR analysis and normalization to the expression of β-actin. Values represent means ± s.d. LIV, liver; WAT, white adipose tissue; BAT, brown adipose tissue; SM, skeletal muscle; H, heart; P, pancreas; K, kidney; INT, intestine; COL; colon; HYP, hypothalamus.

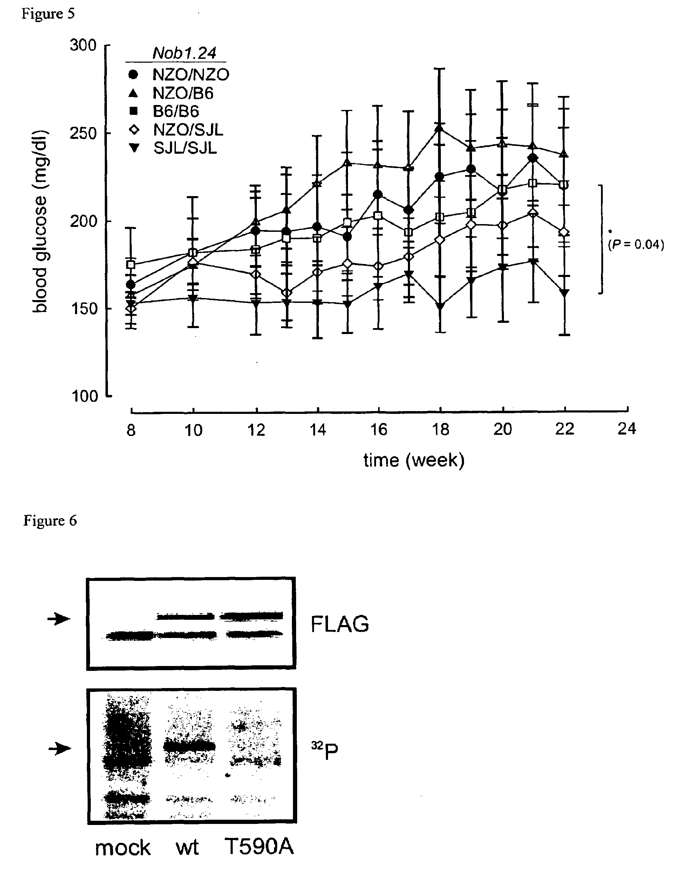

**Figure 5** shows the genotype-dependent blood glucose levels (± s.e.m) of female F2 mice from a reporter cross of RCS B6.SJL-*Nob1*.24 and NZO raised on a HFD (n= 19 - 58). Blood glucose levels are represented as re-transformed geometric means with 95 % confidence interval.
**Figure 6** shows the phosphorylation of T⁵⁹⁰ in *Tbc1d1* by AKT2 kinase *in vitro.* FLAG-tagged wild type *Tbc1d1* and T590A mutant was immunoprecipitated and incubated with purified recombinant AKT2 kinase (∼1 µM) in presence of 100 µM [γ³²-P]ATP for 15 min as described in "Methods". An equal amount of the immunoprecipitate was blotted for the FLAG tag. Phosphate incorporation was visualized by autoradiography. Arrows denote the position of *Tbc1d1.*

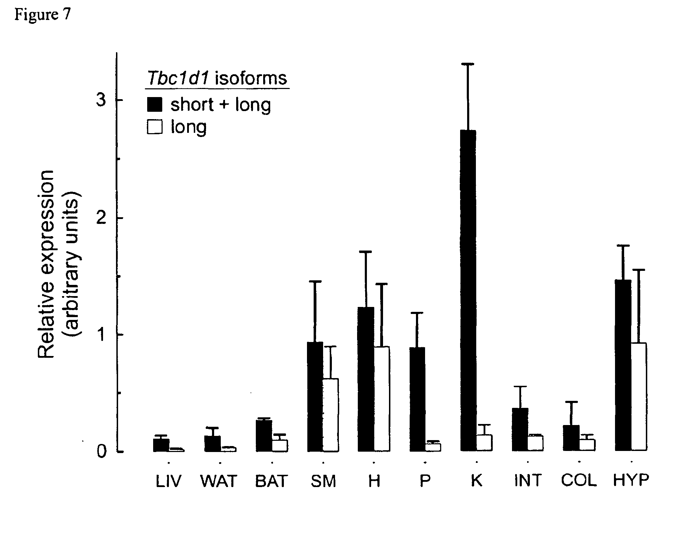

Figure 7 shows the tissue distribution of the two *Tbc1d1* isoforms. Quantification of gene expression was performed with samples from 8 wk old mice (means ± s.d.; n = 5-6) by TaqMan-Realtime-PCR analysis and normalization to the expression of β-actin. LIV, Liver; WAT, white adipose tissue; BAT, brown adipose tissue; SM, skeletal muscle; H, heart; P, pancreas; K, kidney; INT, intestine; COL; colon; HYP, hypothalamus.

Table 1 shows the sequences of primers and probes.

### Examples

In order to identify dietary fat responsive genetic loci in mice as a model for human obesity, the inventors performed crossbreeding experiments of the New Zealand Obese (NZO) mouse and the lean Swiss Jim Lambert (SJL) strain. The NZO strain presents a metabolic syndrome with early onset polygenic obesity, dyslipidemia, hypertension and a type-2 like diabetes mellitus (Crofford, O.B. and Davis, C.K., Jr. Growth Characteristics, Glucose Tolerance and Insulin Sensitivity of New Zealand Obese Mice. Metabolism 14, 271-80 (1965); Leiter, E.H. et al. NIDDM genes in mice: deleterious synergism by both parental genomes contributes to diabetogenic thresholds. Diabetes 47, 1287-95 (1998); Ortlepp, J.R. et al. A metabolic syndrome of hypertension, hyperinsulinaemia and hypercholesterolaemia in the New Zealand obese mouse. Eur J Clin Invest 30, 195-202 (2000); Jurgens, H.S. et al. Hyperphagia, lower body temperature, and reduced running wheel activity precede development of morbid obesity in New Zealand obese mice. Physiol Genomics 25, 234-41 (2006)) Moreover, NZO mice are highly susceptible to weight gain when fed a high-fat diet (HFD), resulting in the development of morbid obesity with fat depots exceeding 40% of total body weight (Jurgens, H.S. et al. Hyperphagia, lower body temperature, and reduced running wheel activity precede development of morbid obesity in New Zealand obese mice. Physiol Genomics 25, 234-41 (2006). Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003). Jurgens, H.S. et al. Development of diabetes in obese, insulin resistant mice: Essential role of dietary carbohydrates in beta-cell destruction. Diabetologia (in press)(2007)). In contrast, the lean SJL strain is resistant to HFD-induced obesity and diabetes (West, D.B., Boozer, C.N., Moody, D.L. and Atkinson, R.L. Dietary obesity in nine inbred mouse strains. Am J Physiol 262, R1025-32 (1992); Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003)) (Figure 1a). The propensity of NZO mice for HFD-induced body weight gain was retained in the NZO x F1 (SJL x NZO) backcross population (Figure 1a). However, whereas backcross mice and parental NZO animals had similar mean body weights on a low fat diet (LFD), the HFD-induced weight gain of the backcross population was diminished (Figure 1a). In a subsequent genome-wide scan of the backcross population, we identified *Nob1,* a major quantitative trait locus (QTL) on chromosome 5 for high-fat diet (HFD)-induced obesity (body weight, body mass index, total body fat; LOD score 7.9; Figure 1b) (Kluge, R. et al. Quantitative trait loci for obesity and insulin resistance (Nob1, Nob2) and their interaction with the leptin receptor allele (LeprA720T/T10441) in New Zealand obese mice. Diabetologia 43, 1565-72 (2000). Plum, L. et al. Type 2 diabetes-like hyperglycemia in a backcross model of NZO and SJL mice: characterization of a susceptibility locus on chromosome 4 and its relation with obesity. Diabetes 49, 1590-6 (2000)). So far, three further adipogenic alleles from NZO have been mapped to chromosomal regions proximal (*Obq11,* 23 Mb; *Obq12,* 51 Mb) and distal13 (D5Mit7, 93 Mb) of *Nob1,* indicating the presence of additional NZO derived adiposity loci on this chromosome. In addition to its interaction with dietary fat8, *Nob1* showed strong epistatic interaction with *Nidd*/*SJL,* a QTL for pancreatic islet cell destruction11: carriers of the NZO allele of *Nob1* exhibited a pronounced acceleration of the development of diabetes when fed a high fat diet (Giesen, K., Plum, L., Kluge, R., Ortlepp, J. and Joost, H.G. Diet-dependent obesity and hypercholesterolemia in the New Zealand obese mouse: identification of a quantitative trait locus for elevated serum cholesterol on the distal mouse chromosome 5. Biochem Biophys Res Commun 304, 812-7 (2003). Plum, L. et al. Characterisation of the mouse diabetes susceptibilty locus Nidd/SJL: islet cell destruction, interaction with the obesity QTL Nob1, and effect of dietary fat. Diabetologia 45, 823-30 (2002)).

In the NZO/SJL backcross population, heterozygous carriers of the SJL allele at *Nob1* and the corresponding homozygous carriers of the NZO allele were not different in body weight when fed a LFD (Figure 1c). In contrast, *Nob1*NZO/NZO animals showed a markedly increased body weight on a HFD compared with the heterozygous Nob1NZO/SJL animals, indicating that the diet-induced weight gain is either increased by the NZO allele or decreased by the SJL allele of *Nob1*. To assess the net effect of the NZO allele of *Nob1,* we conducted additional crossbreeding experiments with NZO and C57BL/6J laboratory mice. However, in the corresponding NZO/C57BL/6J backcross there was no correlation of the *Nob1* genotype and HFD induced body weight gain, and no linkage of diet-induced body weight to chromosome 5, indicating that *Nob1* may represent an obesity-suppressor gene from the lean SJL stain (Figure lc). To validate the obesity-suppressive effect of *Nob1SJL* on a different genetic background and to narrow down the critical region of the *Nob1*-QTL, we generated a recombinant congenic mouse strain (B6.SJL-*Nob1*.24) harbouring a 24 Mb fragment (53.6Mb - 77.5 Mb) of the *Nob1* region from SJL on a C57BL/6J background (Figure 1b).

Introgression of the *Nob1*.24 segment from SJL into a mixed NZO/C57BL/6J background replicated the lean phenotype of SJL (Figure 1d). On a high fat diet, both homozygous and heterozygous carriers of the *Nob1SJL* allele show markedly reduced body weight and blood glucose levels compared to control animals, respectively (Figure 1d and Figure 5). Hence, *Nob1* represents a suppressor for HFD-induced obesity, associated with a 24 Mb segment on chromosome 5 from the lean SJL strain.

For positional cloning of the *Nob1* gene we performed fine mapping of the Nob1.24 segment. Microsatellite marker analysis of the parental strains, NZO and SJL, revealed a complex pattern of polymorphic and non-polymorphic blocks (Figure 2a).

The peak region of the LOD score curve (*D5Mit82 - D5Mit15*) spans about 8 Mb, including a block of ∼3 Mb where the parental strains, NZO and SJL showed microsatellite fragment length polymorphism for five adjacent markers, *DSMit302, DSMit256, DSMit200, D5Mit303,* and *D5Mit290* (Figure 2a). According to NCBI's *Mus musculus* build 36 assembly, a total of seven protein coding genes (*G6pd2, Centd1, 3110047P20Rik, 0610040J01Rik, AA536743, Pgm1, and Tbc1d1*) and one pseudogene (*Cbfa2t2h-ps1*) map to this polymorphic block (Figure 2b). Together with the 12 protein coding genes of the two adjacent proximal and distal non-polymorphic blocks (*Pcdh7, Klf3, Tlr1, Tlr6, 9130005N14Rik, Tmem156, Klhl5, Wdr19, Recc1, Klb, Rpl9, Lias*), a total of 19 genes map into the about 8 Mb *Nob1* peak region (58Mb - 65.7 Mb).

In addition to sequencing the coding regions of these 19 candidate genes, we performed expression profiling of 302 genes in the entire *Nob1* QTL (about 100 genes within or close to the *Nob1.24* segment) with a custom-made macroarray and RNA from various tissues of the two parental strains, including WAT, BAT, skeletal muscle, liver, and hypothalamus (see Methods). Differential gene expression was verified by consecutive quantitative real time PCR. Of all the genes in the *Nob1.24* segment analyzed, only two transcripts were found to be differentially expressed between the two strains in at least one tissue. A gene for a RIKEN cDNA, 2310045A20Rik, which maps close to *D5Mit132*, i.e. 10 Mb proximal to the LOD peak, was up-regulated in brown fat tissue and liver from SJL mice. In contrast, *Tbc1d1* which maps exactly to the polymorphic peak region of the QTL (Figure 2b), was found to be down-regulated 3-fold in skeletal muscle from the lean SJL mice.

Cloning and sequencing of the cDNA for *Tbc1d1* revealed a SJL-specific 7 bp deletion in exon 18 (A⁴⁰⁴⁸ - T⁴⁰⁵⁴ in Genbank Accession no. AK122445; 4048delACTCGCT), which was absent in SJL-related (SWR and FVB) as well as in Non-related mouse strains, including C57BL/6J, NZO, NZB, NON, AKR and SM, respectively (Figure 3a). This deletion results in a frame shift and premature termination of the protein. The inventors confirmed the expression of a truncated protein by *in vitro* translation of wild type and SJL derived cDNA (Figure 3b). *Tbc1d1* (∼192 kbp; 22 exons; Figure 3a) belongs to the tre-2/USP6, BUB2, cdc16 domain family and shows high amino acid sequence homology (∼60 %) to the insulin signalling protein Tbcld4, also known as AS160 (Bernards, A. GAPs galore! A survey of putative Ras superfamily GTPase activating proteins in man and Drosophila. Biochim Biophys Acta 1603, 47-82 (2003); Kane, S. et al. A method to identify serine kinase substrates. Akt phosphorylates a novel adipocyte protein with a Rab GTPase-activating protein (GAP) domain. J Biol Chem 277, 22115-8 (2002)). The >5000 bp mRNA for *Tbc1d1* codes for a about 130 kDa protein containing two N-terminal phosphotyrosine-binding (PTB) domains, and a C-terminal GTPase-activating (Rab GAP) domain (Figure 3a). Analysis of the crystal structure of the catalytic domain of Gyp1p, a GAP for Ypt proteins, the yeast homologues of Rab proteins, and a high degree of sequence conservation in yeast and mammalian TBC proteins suggest a common catalytic mechanism by which TBC-domain GAPs may accelerate GTP hydrolysis by a 'dual-finger' mechanism (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)).

Thus, the SJL-specific 7 bp-deletion represents a loss-of-function (LOF) mutation since the mutated protein lacks most invariant residues of the GTPase-activating TBC domain, including the conserved and catalytically essential glutamine finger (YxQ978) (Pan, X., Eathiraj, S., Munson, M. and Lambright, D.G. TBC-domain GAPs for Rab GTPases accelerate GTP hydrolysis by a dual-finger mechanism. Nature 442, 303-6 (2006)).

Sequence analysis of the other 18 genes in the about 8 Mb *Nob1* peak region (*Pcdh7, G6pd2, Centdl, 3110047P20Rik, 0610040J01Rik, AA536743, Pgm1,Klf3, Tlr1, Tlr6, 9130005N14Rik, Tmem156, Klhl5, Wdr19, Recc1, Klb, Rpl9, Lias)* yielded a total of 13 coding SNPs in 8 genes (7 non-synonymous polymorphisms in 5 genes) but none specific for the SJL strain and/or likely to impair protein function (Table 1).

Very recently, it has been shown that the Rab specificity of the Tbc1d1 GAP domain *in vitro* is identical to that of *Tbc1d4* (AS 160), and that upon insulin stimulation Tbc1d1 becomes phosphorylated at a potential AKT consensus site which is conserved between Tbc1d1 and AS160. In the present study, we used purified recombinant AKT2 from Baculovirus-infected insect cells to confirm that T590 in Tbc1d1, corresponding to T649 in AS160, is a direct target of AKT2 and the only site in Tbc1d1 phosphorylated by this kinase (Figure 6).

In C57BL/6J and NZO mice *Tbc1d1* is expressed mainly in skeletal muscle, heart, pancreas, kidney and hypothalamus, and only low levels of expression were detected in white and brown adipose tissue, liver, intestine and colon (Figure 4a).

However, in the respective tissues from SJL mice the amount of mRNA for *Tbc1d1* was markedly reduced (Figure 4a). Since no sequence variations were detectable in the about 1.5 kb 5'-flanking region of the *Tbc1d1* gene of SJL (Table 1), we assume that mRNA levels of *Tbc1d1* might be reduced by nonsense-mediated mRNA decay (NMD).

In C57BL/6J and NZO mice, we detected two distinct transcripts of *Tbc1d1* that reflect tissue-specific differential splicing of exons 12 and 13. Quantification of the two splice variants which differ by 93 amino acids revealed that the long isoform of *Tbc1d1* is predominantly expressed in muscle and hypothalamus whereas kidney and pancreas express almost exclusively the short isoform (Figure 7).

The inventors next examined whether *Tbc1d1* expression in different tissues is regulated by the fat content of the diet. In skeletal muscle of C57BL6/J and NZO mice, expression of *Tbc1d1* is significantly increased ∼3-fold in response to high fat diet, whereas no significant changes were observed in the other tissues (Figure 4b). As a result, in skeletal muscle from animals raised on a high-fat diet, the levels of *Tbc1d1* mRNA are increased by about an order of magnitude in NZO mice compared to SJL mice, respectively. Nevertheless, expression levels of *Tbc1d1* were similar in NZO mice, C57BL6/J and leptin deficient ob/ob mice (B6.V-Lep^{*ob*/*ob*}), indicating that *Tbc1d1* expression *per se* does not correlate with body weight.

A recent study presented genetic evidence for linkage of a *TBC1D1* variant (R125W; allele frequency of 0.09) to obesity predisposition in selected human families20. Human *TBC1D1* is highly homologous to mouse *Tbc1d1,* maps to 4p15-p14 and is also expressed predominantly in skeletal muscle and heart. The biological function of *Tbc1d1* and its possible involvement in insulin signalling however is still unknown.

Overexpression of Tbc1d1 in 3T3-L1 adipocytes (which contain only minute amounts of the endogenous mRNA) has been shown to inhibit insulin-stimulated GLUT4 translocation. However, diabetes-resistant SJL mice which naturally lack functional Tbc1d1 show normal glucose tolerance which argues against a major role of Tbc1d1 for insulin-regulated glucose uptake (as at least mentioned in Stone et al, see above).

Taking all together, *Tbc1d1* is the only gene in the QTL peak region with an SJL specific mutation. This mutation disrupts the protein sequence, strongly suggesting that the 4048delACTCGCT mutation is the causal defect underlying the *Nob1* QTL.

Furthermore, the genetic interaction of *Nob1* with dietary fat, and its function as an obesity suppressor in SJL mice coincides with the differential expression of *Tbc1d1* in response to HFD. The inventors propose that *Tbc1d1* is involved in a yet unknown pathway that regulates lipid metabolism and energy homeostasis in skeletal muscle.

### Methods

### Animals and animal procedures.

The principles of laboratory animal care (NIH Publication No. 85-23) were followed and the study was approved by the Animal Experimentation Ethics Committee of the State Ministry of Agriculture, Nutrition and Forestry (State of Brandenburg, Germany). SJL (SJL/NBom) and NZO mice (NZO/HlBom) were from the inventors' own colony. C57BL/6J (C57BL/6JCrl) mice were obtained from Charles River Laboratories (Sulzfeld, Germany). The recombinant congenic strain B6.SJL-*Nob1*.24 was generated by 3-fold backcross of SJL mice to the C57BL/6J strain via marker selection for *Nob1* (*D5Mit290*). N2-backcross of C57BL/6cSJL-Nob1 to the NZO strain was performed as a reporter cross to generate obese mouse lines on a polygenic background. After weaning (3 weeks of age), mice received low fat (Altromin, Lage, Germany; No. 1314, with 5 %, 48 %, and 22.5 % fat, carbohydrates, and protein, respectively; 12.5 kJ/g) or high fat (No. C1057, with 16.0 %, 46.8 %, 17.1 % fat, carbohydrates, and protein, respectively; 15.4 kJ/g) rodent chow. Throughout the study, mice had free access to food and water. Three to six mice were kept per cage (Macrolon, type III) in a temperature-controlled room (20 °C, 55±5 % relative humidity) with a 12 h light-dark cycle and lights on at 6 am. Blood glucose was measured weekly under non-fasting conditions in blood samples collected from the tail vein with an Ascensia ELITE® blood glucose meter (Bayer Health Care, Leverkusen, Germany).

### Genotyping and QTL analysis.

DNA was prepared from mice tails with a DNA isolation kit (InViTek, Berlin, Germany). Animals were genotyped for polymorphic microsatellite markers by PCR with oligonucleotide primers obtained from MWG (Ebersberg, Germany), and microsatellite length was determined by non-denaturing polyacrylamide gel electrophoresis (Table 2). Genotyping of SNPs was done by DNA sequencing.

### DNA sequencing and cloning.

The coding regions of candidate genes in the *Nob1* peak region were sequenced after amplification of the corresponding exons (including about 100 bp flanking regions) from genomic DNA with Phusion Polymerase (NEB) or amplification and/or cloning of the cDNAs based on sequence information of NCBI's reference assembly for C57BL/6J build 36 (Table 1). RNA was extracted from flash-frozen tissues using RNeasy-mini columns (Qiagen, Germany) and cDNAs were synthesized from 2 µg of total RNA using the SuperScriptIII first-strand cDNA synthesis kit (Invitrogen) and oligo-dT primers. Sequences were obtained with an ABI 3130x sequencer and sequence analysis was performed by aligning the sequence to the genomic C57BL/6J sequence using SeqScape software 2.5 (Applied Biosystems). All exonic changes were verified by resequencing.

The cDNAs for the long (AK122445) and short (AK154591) isoforms of mouse *Tcbld1* from skeletal muscle and white adipose tissue of C57BL/6J, NZO and SJL mice were amplified using Phusion Polymerase, cloned into pCR2.1-TOPO vector and sequenced. Nucleotide and amino acid positions in *Tbc1d1* refer to the long isoform.

For construction of the Tbc1d1-T590A mutant, the inventors used the Quick-Change method (Stratagene). Resequencing of the (4048delACTCGCT) deletion in exon 18 was performed on the genomic level with the following mouse strains: AKR/J, C57BL/6J, FVB/N, NON/LtJ, NZB/OlaHsd, NZO/HlBom, SJL/NBom, SM/JCrl, and SWR/J.

### Expression profiling and quantitative real time PCR.

Gene expression profiling using custom made cDNA macroarrays was performed as described (Herwig, R., Aanstad, P., Clark, M. and Lehrach, H. Statistical evaluation of differential expression on cDNA nylon arrays with replicated experiments. Nucleic Acids Res 29, E117 (2001)). Briefly, 10 ng purified PCR probes (about 500 bp) derived from 306 cDNAs of the *Nobl* region were immobilized on nylon filter membranes and hybridized with [α-³³P]dCTP labelled cDNA from various tissues of NZO and SJL mice, including BAT, WAT, liver, skeletal muscle, intestine, and hypothalamus. For hybridization, cDNA was synthesized from 10 µg of total RNA using the SuperScriptIII first-strand cDNA synthesis kit and oligo-dT primers. After hybridization the filter membranes were exposed to phosphorimager screens for 14-16 h, and data from 2-3 independent replicates of each hybridization were used to assess expression differences between the two strains. Strain-dependent differential expression was verified by quantitative SYBR Green real time PCR using a 7300 Real-Time PCR System (Applied Biosystems). Measurements of Tbc1d1 expression was performed with TaqMan RT-PCR; Primers (Supplementary Table 2) were obtained from MWG.

### Protein expression and phosphorylation of Tbc1d1.

*Full-lengthTbc1d1* (short isoform) from C57BL/6J and SJL mice was *in vitro* translated using TNT T7 Quick Coupled Transcription/Translation System (Promega) and [35S]methionine (10 Ci/µl; Hartmann Analytics) according to the manufacturer's instruction. For kinase assays, the long form of *Tbc1d1* containing a N-terminal triple FLAG tag was overexpressed in HeLa cells by Lipofectamine (Invitrogen) transfection and immunoprecipitated using a monoclonal FLAG antibody (Sigma). In vitro phosphorylation of FLAGTbc1d1 and the T590A mutant was using recombinant AKT2 kinase was carried out as described 22. Briefly, the substrates were incubated with affinity-purified, activated AKT2 kinase from *Sf9* cells in kinase buffer (50 mmol/l Tris-HCl pH 7.5, 15 mmol/l Mg-acetate, 5 mmol/l glycerophosphate, 0.2 mmol/l sodium vanadate, 1 mmol/l EGTA), 100 µmol/l [γ32P]ATP for 15 min at RT, and the phosphate incorporation into the proteins was determined after SDS-PAGE and Phosphorimager analysis.

**Accession numbers.** GenBank, EntrezID:. *0610040J01 Rik,* 76261; *3110047P20Rik,* 319807; *9130005N14Rik,* 68303; *Aa536743,* 100532; *Centd1,* 212285; *G6pd2,* 14380; *Klb,* 83379; *Klf3,* 16599; *Klhl5,* 71778; *Lias,* 79464; *Pcdh7,* 54216; *Pgml,* 66681; *Recc1"* 19687; *Rpl9,* 20005; *Tbc1d1d,* 57915; *Tlr1,* 21897; *Tlr6,* 21899; *Tmem156,* 243025; *Ugdh,* 22235; *Wdr19,* 213081.

**Table 1:**

| Primers for cloning of *Tbc1d1* cDNA: | | | | |
|---|---|---|---|---|
| Fwd: 5'-TGCGTCGACGAAGCAGAGACTGTGGAGTGG-3' (SEQ ID NO 5); | | | | |
| Rev: 5'-ATCGTATACAACAACCAGGACTGGAAAGG-3' (SEQ ID NO 6). | | | | |
| | | | | |

| Primers and dual labelled probes (5'6-FAM: 3'TAMRA) for expression analysis of *Tbc1d1:* | | | | |
|---|---|---|---|---|
| 1) long and short isoform | | | | |
| Fwd: 5'-AGTGGCCACTCCACAGAAG-3' (SEQ ID NO 7); | | | | |
| Rev: 5'-TCCTGTACTGGGCCAAATG-3' (SEQ ID NO 8); | | | | |
| Probe: 5'-ACTCCCCGAGCAGATATGAAGATTATTCCGAGCTG-3' (SEQ ID NO 9). | | | | |
| | | | | |
| 2) long isoform | | | | |
| Fwd: 5'-ATGTGGACCATCTTCCTGG-3' (SEQ ID NO 10); | | | | |
| Rev: 5'-GCTCTCCACTGGAATTGTG-3' (SEQ ID NO 11); | | | | |
| Probe: 5'TTCAGCTCCGCCTCCACCTCGTCTTAA-3' (SEQ ID NO 12). | | | | |
| | | | | |

| Primers for genotyping the 4048delACTCGCT mutation (product size is 81 bp *vs*. 74 bp): | | | | |
|---|---|---|---|---|
| Fwd: 5'-GAGCAGGTCAGCTGTCACTTT-3' (SEQ ID NO 13) | | | | |
| Rev: 5'-TGAGACCTTGGCAGTATCCA-3' (SEQ ID NO 14) | | | | |

| **Table 1** continued: Microsatellite markers for genotyping of the *Nobl.24* fragment | | | | |
|---|---|---|---|---|
| Marker | genomic position^{a} | polymorphic^{b} | Fwd (5'->3') | Rev (5'->3') |
| D5Mit132 | 53578180 | + | AAAGGTTAAATCTGGATCTTGAGTG | CTCCTCAAGTTTGTTTTGCTCA |
| D5Mit183 | 53625392 | - | TATAAAGATAATCAGGGCTTAAACTCG | ACCTCCACAACATGAGCACA |
| D5Mit133 | 54542016 | - | TTTAAGTTGTGTTAATGTTGAAAGTTC | CACACATGCACGAACATGTA |
| D5Mit395 | 54616504 | - | GCATGTGCGTGTGCTTATG | AATGTATACCTCCACAAACACCTC |
| D5Mit394 | 54631083 | - | TAGATGCTGCCTAACAATTAGTAAGT | TGGGAATATTTTTTCAAGTGTCTC |
| D5Mit300 | 54774683 | - | AGAATTGGGACGAAGCCTTT | TTTGAAATCAAGATTAGCCATTACC |
| D5Mit 301 | 55094495 | - | AAAATCAGTTAAGGTAGCACAATGC | GTTGGCCATCTTTGCTGG |
| D5Mit255 | 55242576 | - | CCCTGTGCTCTGGATTAGTTG | TCAAGACCAGCATCAAACCA |
| D5Mit254 | 55519229 | - | GTGCAGGCCTGAATTGAAAT | CAAAGTGCCTGTGCATGTG |
| D5Mit184 | 56299918 | + | AAGAGAGACATACACAAACAGACACA | TTTCTTAACAAGTTTCTAGCAATTTCC |
| D5Mit199 | 59238877 | - | TGTCTGTCTGTCATTCTGTCATATATG | CCAAAAACCAGATTTGAGATCC |
| D5Mit82 | 59906713 | - | AAGACCACTTGAAAACAGTGCA | GGAGATCTAAATTTCAAAACACCA |
| D5Mit302 | 61716737 | + | CATTTTAAAACAGAAATCTGAAGTTCC | GTGACTAGGTATGCCAAATCCC |
| D5Mit256 | 63251154 | + | TCATGACTATAAATGTATTCATGTGTG | CTTCAAAGACTCCCATATACCCC |
| D5Mit200 | 63827234 | + | CACAGAGAAGAATCTGCGAGC | TTGCAAAGTGTTTTAATCAGTATTTG |
| D5Mit303 | 64375963 | + | GGTTAAGAAAAAGACAGAAAATCTGC | CAGGTTTTTCATAGAGGTGAGATG |
| D5Mit290 | 64701322 | + | ACCCCTATTCAGTGCCAGG | ACCCAGGCCACAAAAGAAC |
| D5Mit197 | 64928223 | - | TCAGTGATAATGACATGGTAGAAGTG | AGAGGGTTTCCTTCCCCC |
| D5Mit15 | 65702109 | + | TCAAAACCCAGCCTCCTG | AGAGTGTGACTAACAGGTGAGGC |
| D5Mit258 | 66321439 | + | CCATCTCTCCAGCCCCTAGA | CTATGGGCACCAGTCAGACA |
| D5Mit110 | 67087462 | + | GGCAACACAAAATGGACTCA | CCAACATCTCCTGTGTCCCT |
| D5Mit234 | 67414397 | + | TCAGTTTGATTGGGTTTCTTCC | CACACATACATGCACTCCTGC |
| D5Mit111 | 68136872 | - | ACCCCATCTCTGTCTCTCTGA | CTGGGTGAGAGGGGAGGT |
| D5Mit257 | 68410547 | + | TTAGCCCACTGTAGCATCACC | TATTTTTCACACAAGAACTTACTGGG |
| D5Mit270 | 68901210 | + | TACCTACCACAATGCTCTGGG | TCCCATTGAGCCTTTTAGGA |
| D5Mit185 | 69745501 | + | CTGAAAAAAAAAGTTCTCTATTCTTGA | TGAGCAGAAAGTCTATTCCAGAA |
| D5Mit83 | 70169646 | + | CATTTTCATCTTATTGCCCATG | ACCTAAAGACAGATACAGGAACCC |
| D5Mit304 | 70428648 | + | ATTATCAGCCATCTTCATTCATACA | GCCTCACCTTTAAACAATACTACTAGA |
| D5Mit305 | 72688283 | - | AAGATGGGAAAATCAGGGATG | AAATGTTCCCTTCATTTTCTTCC |
| D5Mit336 | 72767666 | + | GCATAAAATGGATTTCCTGAGG | TGTCCCAAAACAAACAACCC |
| D5Mit 134 | 73054476 | + | AAGCCAGAAGCCTGTTGTGT | GTCTGTTGTGAGTTCAGTAGGGG |
| D5Mit356 - | 73475143 | - | CCATGCCCAGTCTGGTATCT | TTTTACTTTCCACTAAGCATTCCC |
| D5Mit58 | 73569655 | - | CCTTCCACATACATAGGCAGG | CCTCAGTGCTGGATGGATTT |
| D5Mit135 | 75097410 | + | TACACAGGGAAAGGACAGGG | AGGGAGATTTTGGATTAGAGGC |
| D5Mit201 | 75436384 | + | GAGGACTCCTTCGATTTCCC | TTCCTAAGCAGGAACTGACCA |
| D5Mit235 | 75451538 | - | ACTTCTTGTTACTGCATGTGAAGC | CAAGAGAGCAGAGTTAGTGGCA |
| D5Mit307 | 76281964 | + | AGTGGCATAACTTCATTCAATAATG | GGAATCAAGTTGTTTTTTAAATTTACC |
| a) Chr. 5; NCBI's *Mus musculus* Genome Build 36.1 (May 08, 2006); b) microsatellite fragment length polymorphism for NZO *vs.* SJL strain | | | | |

| **Table 1** continued: Sequencing primers for genotyping of SNP markers in the *Nob1.24* fragment | | | | |
|---|---|---|---|---|
| Marker | SNP-Position | Fwd (5'->3') | | Rev (5'->3') |
| rs3656439 | 45151292 | AGGGCAGCAGCAACAAATAC | | TCATCGTATAAATGTATCAATTCTCAA |
| rs13478239 | 48942621 | AGCTTTTACTGAAATGTTTTGTAGG | | TGAGCAGGAGCCTATTTGTG |
| rs3719870 | 67006506 | CCCTTCAGAGCCAAGAGAAA | | TTCCTTTGCCTCTGTCACCT |
| rs6340166 | 73076177 | CACGCCTCCATTAGAACCTC | | TTTCATGGATCTCTTAGGCACA |
| rs13478334 | 77463162 | CGCTGAATTCTTCCCAAGTG | | GGTCATCTCTAGGCAGGATGTT |
| rs13478357 | 83480774 | GGTCAAGTGAAATCTTCCTGCT | | CCAGTTAAGCATTTCCCCCTA |
| rs13478392 | 92825964 | GCCCTGCAGATAGACGCTTT | | ACCCAAGGTTTTCTGGCTCT |

## Claims

1. A method for diagnosing obesity or diabetes or an increased risk for obesity or diabetes in a mammal, comprising analysis of the biological activity and/or expression of TBC1D1 in a biological sample obtained from said mammal to be diagnosed, wherein a loss of function mutation in the Rab-specific GTP-ase activating TBC domain in the gene for TBC1D1 or a lack or reduced biological activity of TBC1D1 in said mammal is indicative for an absence or a reduced risk for obesity or diabetes in said mammal, wherein said biological activity of TBC1D1 is the activity of the Rab-specific GTP-ase activating TBC domain.

2. The method according to claim 1, wherein said obesity or diabetes is dietary fat-related.

3. The method according to any of claims 1 or 2, wherein said diagnoses comprises the use of TBC1D1-specific antibodies, hybridisation analysis, quantitative PCR, mRNA analysis, quantitative analysis of the amount of TBC1D1-protein, sequencing of the TBC1D1-locus, and/or an analysis of the activity of TBC1D1 in said sample.

4. The method according to any of claims 1 to 3, wherein TBC1D1 is down-regulated at least 3-fold in skeletal muscle.

5. The method according to any of claims 1 to 4, wherein the gene for TBC1D1 contains a 7 bp deletion in exon 18 of the mouse gene for TBC1D1 or the homologous deletion in the human gene for TBC1D1, corresponding to A4048-T4054 of SEQ ID No. 3.

6. A method of identifying a compound that modulates the biological activity of TBC1D1 in a cell, comprising the steps of
a) contacting a cell expressing TBC1D1 with at least one potentially interacting compound, and
b) measuring a lack or reduced biological activity of TBC1D1 in said cell, wherein said biological activity of TBC1D1 is the activity of the Rab-specific GTP-ase activating TBC domain.

7. The method according to claim 6, wherein said compound as identified is an inhibitor of the biological activity of TBC1D1 in said cell.

8. The method according to claim 7, wherein said compound is selected from a peptide library, a combinatory library, a cell extract, in particular a plant cell extract, a "small molecular drug", an antibody or fragment thereof, an antisense oligonucleotide, an siRNA, a protein and/or a protein fragment, in particular an antibody recognizing the amino acid sequence TGQPSAPGPRPMRKS.

9. Use of a cell that is transformed with an expression vector comprising a polynucleotide encoding a polypeptide selected from
a) a polypeptide that is encoded by SEQ ID NO: 3 or 4,
b) a polypeptide having an amino acid sequence having a 90% or more homology with an amino acid sequence of SEQ ID NO: 1 or 2, and exhibiting a TBC1D1 activity,
c) a polypeptide having an amino acid sequence in which 1 to 10 amino acids are deleted, substituted, and/or added in the amino acid sequence according to SEQ ID NO: 1 or 2, and exhibiting a TBC1D1 activity, and
d) a polypeptide consisting of an amino acid sequence of SEQ ID NO: 1 or 2,
wherein said cell expresses said polypeptide, or a non-human transgenic mammal over-expressing TBC1D1 and/or carrying a TBC1D1 genetic reporter-construct,
wherein said biological activity of TBC1D1 is the activity of the Rab-specific GTP-ase activating TBC domain
as a screening tool in a method for screening for an agent for treating or preventing obesity or diabetes according to any of claims 6 to 8.

## Patentansprüche

1. Verfahren zur Diagnose von Fettsucht oder Diabetes oder eines erhöhten Risikos für Fettsucht oder Diabetes in einem Säuger, umfassend die Analyse der biologische Aktivität und/oder Expression von TBC1D1 in einer biologischen Probe, die von dem zu diagnostizierenden Säuger erhalten wurde, wobei eine funktionszerstörende Mutation in der Rab-spezifischen GTP-ase aktivierenden TBC Domäne in dem Gen für TBC1D1 oder ein Fehlen oder eine reduzierte biologische Aktivität von TBC1D1 in dem Säuger ein Fehlen oder ein reduziertes Risiko für Fettsucht oder Diabetes in dem Säuger anzeigt, wobei die biologische Aktivität von TBC1D1 die Aktivität der Rab-spezifischen GTP-ase aktivierenden TBC Domäne ist.

2. Verfahren nach Anspruch 1, wobei die Fettsucht oder Diabetes mit dem Emährungsfett zusammenhängt.

3. Verfahren nach einem der Ansprüche 1 oder 2, wobei die Diagnose die Verwendung von TBC1D1-spezifischen Antikörpern, Hybridisierungsanalyse, quantitative PCR, mRNA Analyse, quantitative Analyse der Menge an TBC1D1-Protein, Sequenzieren des TBC1D1-Lokus und/oder eine Analyse der Aktivität von TBC1D1 in der Probe umfasst.

4. Verfahren nach einem der Ansprüche 1 bis 3, wobei TBC1D1 im Skelettmuskel mindestens 3-fach herunter reguliert ist.

5. Verfahren nach einem der Ansprüche 1 bis 4, wobei das Gen für TBC1D1 eine 7 bp Deletion in Exon 18 des Maus-Gens für TBC1D1 oder die homologe Deletion im menschlichen Gen für TBC1D1, entsprechend zu A4048-T4054 von SEQ ID No. 3, enthält.

6. Verfahren zur Identifizierung einer Verbindung, die die biologische Aktivität von TBC1D1 in einer Zelle moduliert, umfassend die Schritte von
a) in Kontakt bringen einer Zelle, die TBC1D1 exprimiert, mit mindestens einer potentiell interagierenden Verbindung, und
b) Messen eines Fehlens oder einer reduzierten biologische Aktivität von TBC1D1 in der Zelle, wobei die biologische Aktivität von TBC1D1 die Aktivität der Rab-spezifischen GTP-ase aktivierenden TBC Domäne ist.

7. Verfahren nach Anspruch 6, wobei die Verbindung wie identifiziert ein Inhibitor der biologischen Aktivität von TBC1D1 in der Zelle ist.

8. Verfahren nach Anspruch 7, wobei die Verbindung ausgewählt ist aus einer Peptid-Bibliothek, einer kombinatorischen Bibliothek, einem Zellextrakt, insbesondere einem Pflanzenzellextrakt, einem "Wirkstoff mit kleinem Molekulargewicht", einem Antikörper oder Fragment davon, einem antisense Oligonukleotid, einer siRNA, einem Protein und/oder einem Proteinfragment, insbesondere einem Antikörper, der die Aminosäuresequenz TGQPSAPGPRPMRKS erkennt.

9. Verwendung einer Zelle, die mit einem Expressionsvektor transformiert ist, umfassend ein Polynukleotid, das für ein Polypeptid kodiert, ausgewählt aus
a) einem Polypeptid, das durch SEQ ID NO: 3 oder 4 kodiert wird,
b) einem Polypeptid, das eine Aminosäuresequenz aufweist, die 90% oder mehr Homologie mit einer Aminosäuresequenz von SEQ ID NO: 1 oder 2 aufweist, und das eine TBC1D1 Aktivität zeigt,
c) einem Polypeptid, das eine Aminosäuresequenz aufweist, in der 1 bis 10 Aminosäuren deletiert, substituiert und/oder zu der Aminosäuresequenz nach SEQ ID NO: 1 oder 2 hinzugefügt sind, und das eine TBC1D1 Aktivität zeigt, und
d) einem Polypeptid bestehend aus einer Aminosäuresequenz von SEQ ID NO: 1 oder 2,
wobei die Zelle das Polypeptid exprimiert, oder ein nicht-menschliches transgenes Säugertier, das TBC1D1 überexprimiert und/oder ein TBC1D1 genetisches Reporterkonstrukt trägt,
wobei die biologische Aktivität von TBC1D1 die Aktivität der Rab-spezifischen GTP-ase aktivierenden TBC Domäne ist
als ein Screeningtool in einem Verfahren zum Screening auf ein Mittel zur Behandlung oder der Prävention von Fettsucht oder Diabetes nach einem der Ansprüche 6 bis 8.

## Revendications

1. Procédé pour le diagnostic de l'obésité ou du diabète ou d'un risque accru de l'obésité ou du diabète chez un mammifère, comprenant une analyse de l'activité biologique et/ou de l'expression de TBC1D1 dans un échantillon biologique obtenu à partir dudit mammifère à diagnostiquer, dans lequel une mutation de perte de fonction dans le domaine TBC d'activation de GTP-ase spécifique à Rab dans le gène codant pour TBC1D1 ou une absence ou une réduction de l'activité biologique de TBC1D1 chez ledit mammifère indique une absence ou une réduction de risque de l'obésité ou du diabète chez ledit mammifère, dans lequel ladite activité biologique de TBC1D1 est l'activité du domaine TBC d'activation de GTP-ase spécifique à Rab.

2. Procédé selon la revendication 1, dans lequel ladite obésité ou ledit diabète est lié(e) aux graisses alimentaires.

3. Procédé selon l'une des revendications 1 ou 2, dans lequel ledit diagnostic comprend l'utilisation d'anticorps spécifiques à TBC1D1, une analyse par hybridation, une PCR quantitative, une analyse de l'ARNm, une analyse quantitative de la quantité de protéine TBC1D1, un séquençage du locus de TBC1D1, et/ou une analyse de l'activité de TBC1D1 dans ledit échantillon.

4. Procédé selon l'une des revendications 1 à 3, dans lequel TBC1D1 est régulé négativement au moins d'un facteur 3 dans le muscle squelettique.

5. Procédé selon l'une des revendications 1 à 4, dans lequel le gène codant pour TBC1D1 contient une délétion de 7 pb dans l'exon 18 du gène de souris codant pour TBC1D1 ou la délétion homologue dans le gène humain codant pour TBC1D1, correspondant à A4048-T4054 de la séquence SEQ ID No.3.

6. Procédé d'identification d'un composé qui module l'activité biologique de TBC1D1 dans une cellule, comprenant les étapes qui consistent
a) à mettre en contact une cellule exprimant TBC1D1 avec au moins un composé interagissant potentiellement, et
b) à mesurer une absence ou une réduction de l'activité biologique de TBC1D1 dans ladite cellule, où ladite activité biologique de TBC1D1 est l'activité du domaine TBC d'activation de GTP-ase spécifique à Rab.

7. Procédé selon la revendication 6, dans lequel ledit composé tel qu'identifié est un inhibiteur de l'activité biologique de TBC1D1 dans ladite cellule.

8. Procédé selon la revendication 7, dans lequel ledit composé est choisi à partir d'une banque de peptides, d'une banque combinatoire, d'un extrait de cellules, en particulier un extrait de cellules végétales, d'un « médicament de faible poids moléculaire », d'un anticorps ou d'un fragment de celui-ci, d'un oligonucléotide antisens, d'un ARNsi, d'une protéine et/ou d'un fragment protéique, en particulier un anticorps reconnaissant la séquence d'acides aminés TGQPSAPGPRPMRKS.

9. Utilisation d'une cellule qui est transformée par un vecteur d'expression comprenant un polynucléotide codant pour un polypeptide choisi parmi
a) un polypeptide qui est codé par la séquence SEQ ID NO : 3 ou 4,
b) un polypeptide ayant une séquence d'acides aminés ayant une homologie de 90% ou plus avec une séquence d'acides aminés de la SEQ ID NO : 1 ou 2, et présentant une activité de TBC1D1,
c) un polypeptide ayant une séquence d'acides aminés dans laquelle de 1 à 10 acide(s) aminé(s) est/sont délété(s), substitué(s), et/ou ajouté(s) dans la séquence d'acides aminés selon la séquence SEQ ID NO : 1 ou 2, et présentant une activité de TBC1D1, et
d) un polypeptide constitué d'une séquence d'acides aminés de la SEQ ID NO _{:} 1 ou 2,
où ladite cellule exprime ledit polypeptide, ou un mammifère transgénique non-humain sur-exprimant TBC1D1 et/ou portant une construction de rapporteur génétique de TBC1D1,
où ladite activité biologique de TBC1D1 est l'activité du domaine TBC d'activation de GTP-ase spécifique à Rab en tant qu'outil de criblage dans un procédé de criblage pour un agent destiné à traiter ou à prévenir l'obésité ou le diabète selon l'une des revendications 6 à 8.
